# EUROPÄISCHE PATENTSCHRIFT

(11) **EP 0 646 580 B1**
(45) Veröffentlichungstag und Bekanntmachung des Hinweises auf die Patenterteilung: **31.05.2000**
(21) Anmeldenummer: 94810517.6
(22) Anmeldetag: 07.09.1994
(51) Int. Cl.: C07D 303/28, C07D 303/40, C07C 271/18, C07C 271/38, G03C 9/08, G03F 7/028, C08F 2/48

(54) **Vinyletherverbindungen mit zusätzlichen von Vinylethergruppen verschiedenen funktionellen Gruppen und deren Verwendung zur Formulierung härtbarer Zusammensetzungen**
Vinylether compounds with additional functional groups differing from vinylether and their use in the formulation of curable compositions
Composés de vinyléther avec des groupes fonctionnels additionnels différents de vinyléther et leur utilisation dans la formulation de compositions durcissables

(30) Priorität: 16.09.1993 CH 278693; 08.03.1994 CH 68494
(43) Veröffentlichungstag der Anmeldung: 05.04.1995
(73) Patentinhaber: Ciba Specialty Chemicals Holding Inc., 4057 Basel (CH)
(72) Erfinder: Steinmann, Bettina, Dr., CH-1724 Praroman (CH); Schulthess, Adrian, Dr., D-1734 Tentlingen (CH); Hunziker, Max, Dr., CH-3186 Düdingen (CH)

(56) Entgegenhaltungen:
- WO-A-89/03816
- WO-A-91/05814
- WO-A-92/20014
- DE-B- 1 027 401
- FR-A- 2 270 269
- GB-A- 1 436 443
- JP-A- 4 195 059
- US-A- 2 692 256
- US-A- 4 098 918
- CHEMICAL ABSTRACTS, vol. 80, no. 20, 1974, Columbus, Ohio, US; abstract no. 109994c, & JP-A-48 075 637 (NIPPON OIL SEAL INDUSTRY CO LTD) & REGISTRY (Datenbank, STN) RN: 41440-38-4
- CHEMICAL ABSTRACTS, vol. 83, no. 20, 1975, Columbus, Ohio, US; abstract no. 164616v, & VYSOKOMOL. SOEDIN., SER. B, Bd.17, Nr.3, 1975 G.A. VOSKOBOINIK ET AL & REGISTRY (Datenbank, STN) RN: 56793-09-0
- CHEMICAL ABSTRACTS, vol. 101, no. 20, 1984, Columbus, Ohio, US; abstract no. 172546c, & RADIATS. KHIMIYA I TEKHNOL. OLIGOMER. SISTEM, M., 1983 Seiten 62 - 69 V.S. IVANOV ET AL & REGISTRY (Datenbank, STN) RN: 92641-21-9
- CHEMICAL ABSTRACTS, vol. 121, no. 2, 1994, Columbus, Ohio, US; abstract no. 11927a, & JP-A-06 016 731 (NIPPON KAYAKU KK) 25. Januar 1994 & REGISTRY (STN, Datenbank) RN: 155757-49-6, 155757-50-9
- CHEMICAL ABSTRACTS, vol. 120, no. 10, 1994, Columbus, Ohio, US; abstract no. 120749u, & SU-A-1 412 491 (UKRAINIAN PRINTING INSTITUTE) & REGISTRY (STN, Datenbank) RN: 1464-69-3
- CHEMICAL ABSTRACTS, vol. 118, no. 12, 1993, Columbus, Ohio, US; abstract no. 113055v, & JP-A-04 195 059 (KONICA CO) & REGISTRY (Datenbank, STN) RN:146248-84-2
- CHEMICAL ABSTRACTS, vol. 114, no. 12, 1991, Columbus, Ohio, US; abstract no. 103626k, & JP-A-02 242 820 (TOSOH CORP) & REGISTRY (Datenbank, STN) RN: 132429-77-7, 132429-88-0
- CHEMICAL ABSTRACTS, vol. 115, no. 8, 1991, Columbus, Ohio, US; abstract no. 73736x, & JP-A-03 037 229 (KANSAI PAINT CO LTD) & REGISTRY (Datenbank, STN) RN: 135408-44-5
- CHEMICAL ABSTRACTS, vol. 115, no. 8, 1991, Columbus, Ohio, US; abstract no. 73713n, & JP-A-02 233 717 (KANSAI PAINT CO LTD) & REGISTRY (Datenbank, STN) RN: 131718-57-5
- CHEMICAL ABSTRACTS, vol. 93, no. 11, 1980, Columbus, Ohio, US; abstract no. 114308g, & SU-A-727 644 (IRKUTSK INSTITUTE OF ORGANIC CHEMISTRY) & REGISTRY (Datenbank, STN) RN: 74802-05-4, 74802-06-5, 74802-07-6
- CHEMICAL ABSTRACTS, vol. 68, no. 9, 1968, Columbus, Ohio, US; abstract no. 39104r, & SU-A-196 771 (IRKUTSK INSTITUTE OF ORGANIC CHEMISTRY) & REGISTRY (Datenbank, STN) RN: 18381-33-4, 16801-19-7, 4587-01-3
- TETRAHEDRON LETT., Bd.25, Nr.2, 1984 Seiten 123 - 126 F.E. ZIEGLER ET AL
- CHEMICAL ABSTRACTS, vol. 120, no. 24, 1994, Columbus, Ohio, US; abstract no. 300219e, & JP-A-05 310 811 (NIPPON KAYAKU KK) 22. November 1993 & REGISTRY (Datenbank, STN) RN: 154881-78-4, 154881-80-8, 154881-82-0
- CHEMICAL ABSTRACTS, vol. 93, no. 2, 1980, Columbus, Ohio, US; abstract no. 16985f, & JP-A-54 161 319 (SOMAR MFG CO LTD) & REGISTRY (Datenbank, STN) RN: 73946-79-9
- MAKROMOL. CHEM., RAPID COMMUN., Bd.12, Nr.7, 1991 Seiten 447 - 453 M. SCHAPPACHER ET AL
- J. POLYM.SCI., POLYM. CHEM. ED., Bd.16, Nr.4, 1978 Seiten 1367 - 1373 R.E. THOMPSON ET AL

## Beschreibung

Die Erfindung betrifft neue Vinyletherverbindungen, deren Verwendung und Zusammensetzungen, die diese Verbindungen enthalten, sowie weiterhin die Verwendung dieser Zusammensetzungen zur Erzeugung gehärteter Produkte, insbesondere nach dem stereolithographischen Verfahren.

Es ist seit langem bekannt, Verbindungen mit Kohlenstoffdoppelbindungen als polymerisierbaren Bestandteil härtbarer Zusammensetzungen zu verwenden, unter anderem auch Polyvinyletherverbindungen. In speziellen Fällen kann es hierbei erwünscht sein, die Härtung einer polymerisierbaren Zusammensetzung stufenweise durchzuführen. So sind z. B. in der WO 92/20014 polymerisierbare Zusammensetzungen beschrieben, die neben einer Vinyletherverbindung eine Epoxidverbindung als weiteren polymersierbaren Bestandteil enthalten. Mit Hilfe dieser Zusammensetzungen ist es möglich, stereolithographisch Formkörper besonders hoher Formtreue herzustellen.

Zusammensetzungen, wie die beschriebene, haben jedoch noch Nachteile. So sind sie z. B. nur relativ wenig strahlungsempfindlich. Weiterhin können die mechanischen Eigenschaften, insbesondere der Elastizitätsmodul (E-Modul) und die Reissdehnung von Materialien, die mit Hilfe derartiger Zusammensetzungen erhalten wurden, oftmals nicht befriedigen. Der vorliegenden Erfindung liegt daher die Aufgabe zugrunde, zu weiteren Verbesserungen hinsichtlich der genannten nachteiligen Eigenschaften der in Rede stehenden Zusammensetzungen zu gelangen. Überraschenderweise hat es sich gezeigt, dass bei Verwendung von Vinyletherverbindungen als einer polymerisierbaren Komponente strahlungshärtbare Zusammensetzungen grosser Empfindlichkeit erhalten werden können, die zudem im ausgehärteten Zustand ein homogenes Material hoher Netzwerkdichte und von hohem E-Modul sowie guter Reissfestigkeit ergeben, wenn die Vinyletherverbindungen ausser den Vinylethergruppen von diesen verschiedene funktionelle Gruppen im Molekül enthalten, die zu einer Vernetzungsreaktion fähig sind, wie Acrylat-, Methacrylat-, Epoxid-, Alkenyl-, insbesondere Vinylalkyl-, Cycloalkenyl-sowie Vinylaryl-, insbesondere Styrylgruppen.

Die Erfindung betrifft daher Verbindungen mit mindestens einer Vinylethergruppe, die ausserdem mindestens eine weitere funktionelle Gruppe ausgewählt aus Acrylat-, Methacrylat-, Epoxid-, Alkenyl-, Cycloalkenyl- sowie Vinylarylgruppen im Molekül aufweisen, dadurch gekennzeichnet, dass es sich um Verbindungen der folgenden Formel handelt:

[H₂C = CH―O⁆_{z}A

wobei die in dieser und den weiteren Formeln verwendeten Symbole die nachfolgende Bedeutung haben:
- A: ein z-wertiger Rest ausgewählt aus den Resten der nachfolgenden Formeln (1), (2), (3) und (4) 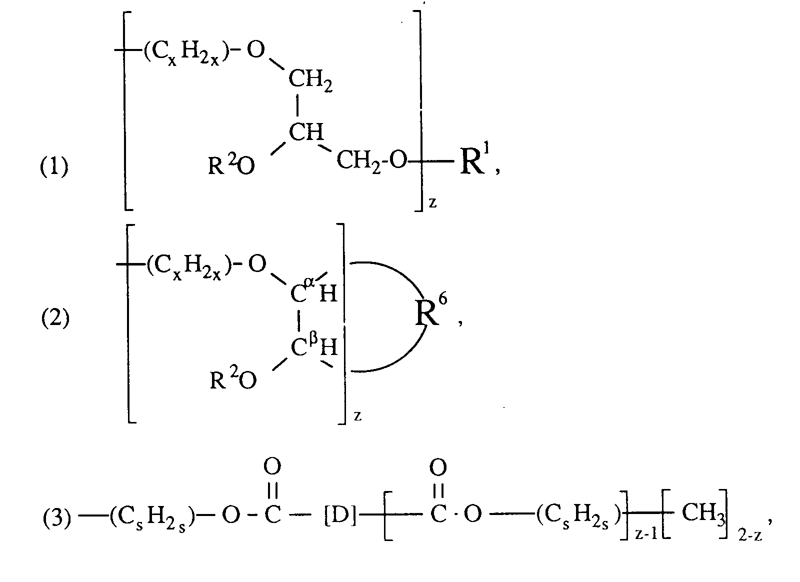 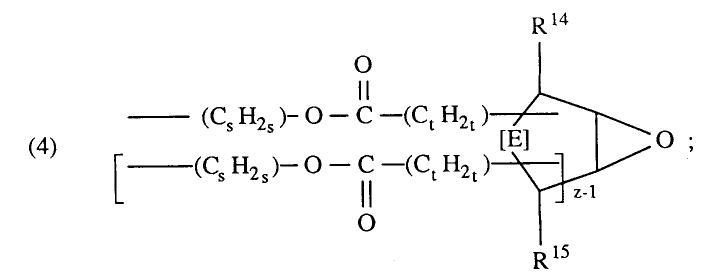
- [D]: eine Gruppe der Formel 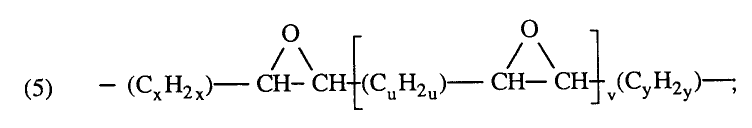
- [E]: eine C₁- oder eine C₂-Alkylengruppe;
- R⁰: ein Wasserstoffatom oder eine Methylgruppe;
- R¹: ein z-wertiger Rest ausgewählt aus aliphatischen, cycloaliphatischen, aromatischen, aliphatisch-aromatischen und aliphatisch-cycloaliphatischen Resten sowie Polyoxyalkylenresten;
- R²: ein Rest ausgewählt aus den Resten der Formeln 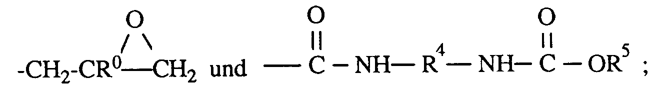
- R⁴: eine Gruppe ausgewählt aus den Gruppen der Formeln 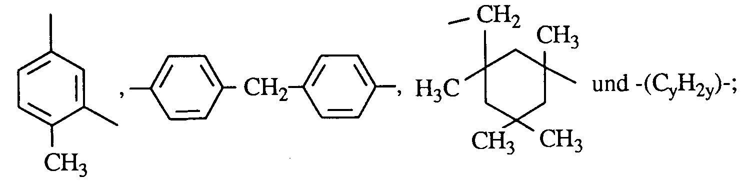
- R⁵: eine Gruppe ausgewählt aus den Gruppen der Formeln 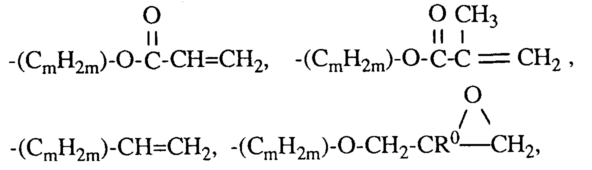 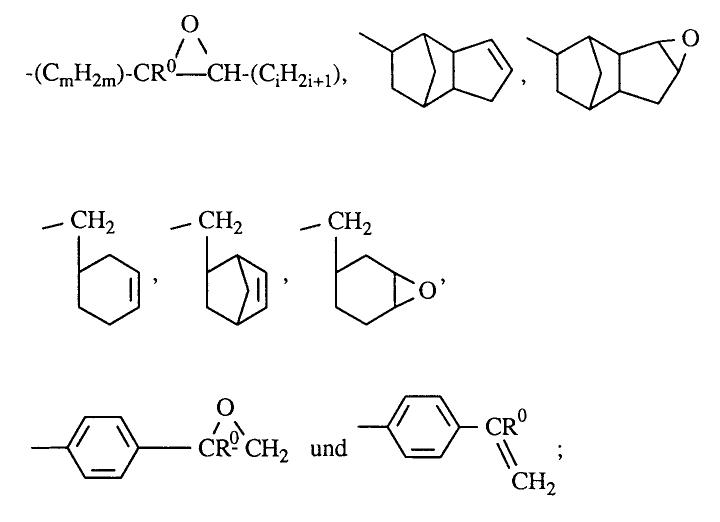
- R⁶: eine (2·z)-wertige organische Gruppe, die jeweils mit den Kohlenstoffatomen C^{α} und C^{β} jeder der z Gruppen der Formel 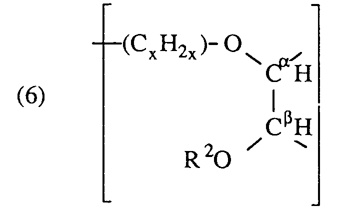 in einem Rest der Formel (2) einen cycloaliphatischen Ring mit mindestens 5 Kohlenstoffatomen bildet;
- R¹⁴ und R¹⁵: jeweils ein Wasserstoffatom oder, falls [E] eine C₂-Alkylengruppe bedeutet, jeweils ein Wasserstoffatom oder zusammen eine Methylengruppe;
- i: eine ganze Zahl von 0 bis 20;
- m: eine ganze Zahl von 1 bis 20;
- s: eine ganze Zahl von 2 bis 10;
- t: eine ganze Zahl von 0 bis 10;
- u: in den einzelnen Einheiten der Formel 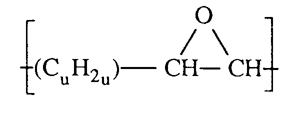 unabhängig voneinander jeweils eine ganze Zahl von 1 bis 20;
- v: eine ganze Zahl von 0 bis 4;
- x: sowie
- y: unabhängig voneinander jeweils eine ganze Zahl von 2 bis 20 und
- z: die Zahl 1 oder 2.

Die erfindungsgemässen Verbindungen, wo A einen Rest der Formel (1) oder (2) darstellt, weisen bevorzugt eine der Formeln 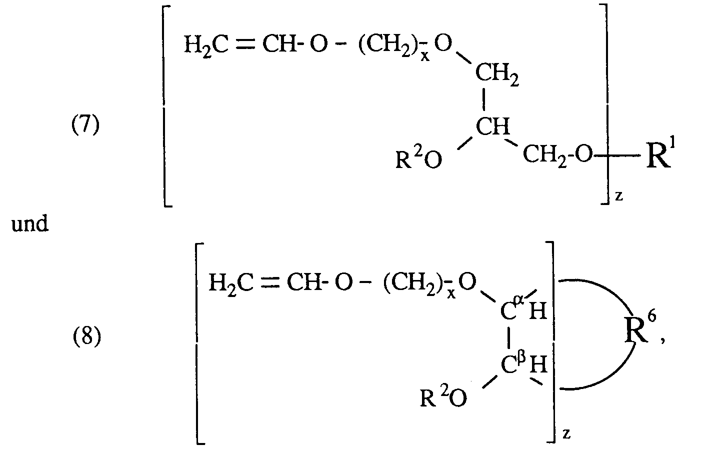 auf, worin R¹, R², R⁶, x, z sowie die übrigen Symbole die oben genannte Bedeutung haben.

Die Indices i, m, s, t, u, x und y weisen zweckmässig eine Obergrenze von 8, die Indices i, m, s, t und u besonders zweckmässig von 6 oder 4 auf.
R⁰ stellt bevorzugt ein Wasserstoffatom und
R² bevorzugt 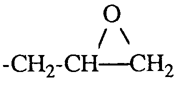 dar.

In den erfindungsgemässen Verbindungen, stellt
- R¹: vorzugsweise einen z-wertigen Rest dar, ausgewählt aus
a) aliphatischen Resten mit 2 bis 20 Kohlenstoffatomen,
b) cycloaliphatischen sowie aromatischen Resten mit jeweils 6 bis 14 Kohlenstoffatomen,
c) aliphatisch-aromatischen sowie aliphatisch-cycloaliphatischen Resten mit jeweils 7 bis 25 Kohlenstoffatomen und
d) Polyoxyalkylenresten der Formeln

   R⁷-[OC_{g}H_{2g}]ₙ- und -(C_{g}H_{2g})-[OC_{g}H_{2g}]ₙ₋₁-,

   worin
   R⁷ eine Alkylgruppe mit 1 bis 8 Kohlenstoffatomen,
   g eine Zahl von 1 bis 8 entsprechend der durchschnittlichen Anzahl von Kohlenstoffatomen einer Alkyleneinheit des Polyoxyalkylenrestes und
   n eine ganze Zahl von 2 bis 20 bedeuten.

Das Symbol "g" gibt die Anzahl von Kohlenstoffatomen an, die sich durchschnittlich für eine Alkyleneinheit in der entsprechenden Polyoxyalkylengruppe errechnet. Der Index g muss also nicht unbedingt ganzzahlig sein, da die Polyoxyalkylengruppe auch aus Monomeren mit unterschiedlicher Anzahl von Kohlenstoffatomen gebildet sein kann, die zudem in unterschiedlichen Verhältnissen eingesetzt sein können. Beispiele für mögliche Monomere sind Ethylenoxid, Propylenoxid oder Tetrahydrofuran. Bevorzugt hat g den Wert 2 oder 3 oder einen Wert zwischen 2 und 3 auf, stellt also einen aus Ethylenoxid-und/oder Propylenoxideinheiten aufgebauten Polyether dar.
- R¹: kann unsubstituiert sein oder zusätzlich einen oder mehrere Substituenten aufweisen, die im Falle eines aliphatischen Restes R¹ ausgewählt sind aus C₁-C₄-Alkoxy- und Halogen-Substituenten; und im Falle der andersartigen Reste R¹ausgewählt aus C₁-C₄-Alkyl-, C₁-C₄-Alkoxy- und Halogen-Substituenten.
- R¹: stellt besonders bevorzugt einen Rest dar ausgewählt aus den Resten der Formeln 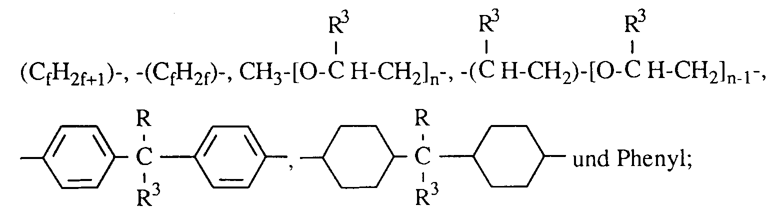 wobei
- R und R³: unabhängig voneinander jeweils ein Wasserstoffatom oder Methyl und
- f: eine ganze Zahl von 2 bis 20 bedeuten.
- n: hat wiederum die oben schon genannte Bedeutung einer ganzen Zahl von 2 bis 20, bevorzugt von 2 bis 10.

Ganz besonders bevorzugt werden schliesslich die Verbindungen der Formel (7), bei denen
- R¹: für einen Rest ausgewählt aus den Resten der Formeln 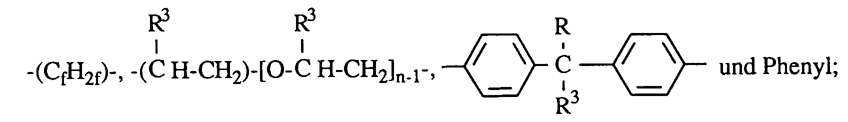
- R⁴: für eine Gruppe ausgewählt aus den Gruppen der Formeln 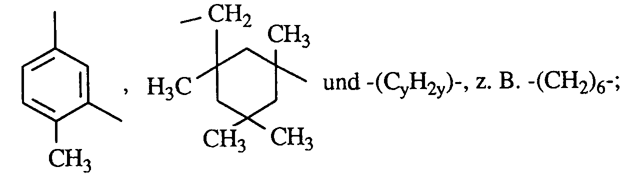
- R⁵: für eine Gruppe ausgewählt aus den Gruppen der Formeln 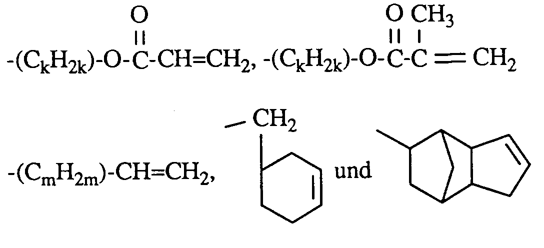 stehen sowie
- R und R³: beide entweder ein Wasserstoffatom oder Methyl; und
- k: eine ganze Zahl von 2 bis 10 bedeuten, für den Index
- m: eine Obergrenze von 10 und für die übrigen Symbole das oben schon Gesagte gilt.

Beispiele solcher Verbindungen sind die, bei denen
- R¹: einen Rest ausgewählt aus einem Alkylenrest mit 2 bis 4 Kohlenstoffatomen, einem Phenylrest und einem Rest der Formel 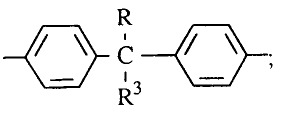
- R⁴: eine Gruppe der Formel 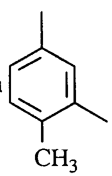 und
- R⁵: eine Gruppe ausgewählt aus den Gruppen der Formeln 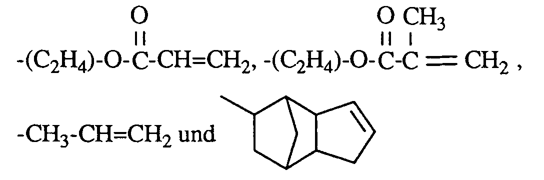 darstellen.

Die erfindungsgemässen Vinyletherverbindungen haben vorzugsweise ein Molekulargewicht unter 3000 und sind ebenfalls vorzugsweise bei Raumtemperatur, d.h. bei ca. 10 bis 30°, fliessfähig

Die Vinyletherverbindungen, bei denen A einen Rest der Formel (1) darstellt, beispielsweise also die Verbindungen der Formel (7), können z. B. in der Weise hergestellt werden, dass man zunächst den Glycidylether 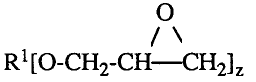 mit einem Hydroxyalkylvinylether der Formel H₂C=CH-O-(CₓH₂ₓ)-OH zu einer Verbindung der Formel 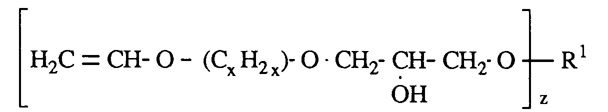 umsetzt und entweder die Hydroxylgruppe in eine Glycidylethergruppe überführt oder mit dem Diisocyanat der Formel OCN-R⁴-NCO und der Hydroxylverbindung R⁵OH beziehungsweise einem Addukt dieser beiden Reaktanten weiterreagieren lässt.

Die Verbindungen, bei denen A einen Rest der Formel (2) darstellt, z. B. die Verbindungen der Formel (8), weisen zumindest einen Cycloalkylrest auf, der von den beiden Kohlenstoffatomen C^{α}und C^{β} eines Strukturelementes der Formel 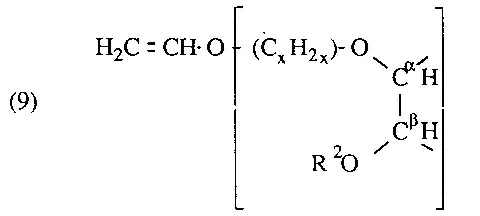 und dem Rest R⁶ insgesamt oder von den beiden Kohlenstoffatomen C^{α} und C^{β} eines Strukturelementes der Formel (9) und Teilen des Restes R⁶ gebildet wird. Unter dem Begriff "Cycloalkylgruppe" werden hierbei insbesondere auch Polycycloalkylreste, z. B. Bicycloalkylreste, verstanden. Ebenso sollen von dem Begriff Cycloalkylgruppen umfasst sein, die aus zwei spiroverknüpften Ringen bestehen. Bevorzugt stellen
- R⁶: eine organische Gruppe mit 3 bis 50, insbesondere mit 3 bis 30 und besonders bevorzugt mit 3 bis 20 Kohlenstoffatomen und
- R²: ein Rest ausgewählt aus den Resten der Formeln 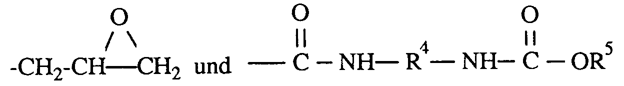 dar, wobei
- R⁴: für eine Gruppe ausgewählt aus den Gruppen der Formeln 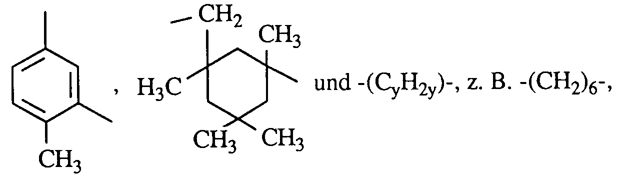 und
- R⁵: für eine Gruppe ausgewählt aus den Gruppen der Formeln 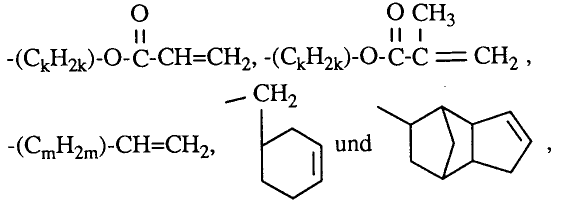
- k: für eine ganze von 2 bis 10 und
- m: für eine ganze Zahl von 1 bis 10 stehen.

Die erfindungsgemässen Verbindungen, bei denen A eine Gruppe der Formel 2 darstellt, umfassen beispielsweise Verbindungen, bei denen
- R⁶: zusammen mit den Kohlenstoffatomen C^{α} und C^{β} der Gruppe der Formel (9) einen Cycloalkylrest mit 5 bis 7 Ringkohlenstoffatomen, insbesondere einen Cyclopentyl-oder Cyclohexylrest, bildet und z den Wert 1 hat.

Weiterhin umfassen die erfindungsgemässen Verbindungen, bei denen A eine Gruppe der Formel 2 darstellt, Verbindungen eines Strukturtyps, bei dem
- R⁶: eine Gruppe der Formel R⁸-[G]-R⁹ darstellt, worin
- R⁸: zusammen mit den Kohlenstoffatomen C^{α} und C^{β} einer Gruppe der oben schon genannten Formel (9) einen Cycloalkylrest mit 5 bis 7 Ringkohlenstoffatomen, insbesondere einen Cyclopentyl- oder Cyclohexylring bildet, an den gegebenenfalls ein weiterer Cycloalkylrest mit 5 bis 7 Ringkohlenstoffatomen annelliert ist;
- R⁹: entweder ebenfalls mit den Kohlenstoffatomen C^{α} und C^{β} einer weiteren Gruppe der Formel (9) einen Cycloalkylrest mit 5 bis 7 Ringkohlenstoffatomen insbesondere einen Cyclopentyl- oder Cyclohexylring bildet, an den gegebenenfalls ein weiterer Cycloalkylrest mit 5 bis 7 Ringkohlenstoffatomen annelliert ist, oder selbst ein Cycloalkylrest mit 5 bis 7 Ringkohlenstoffatomen, insbesondere ein Cyclopentyl- oder Cyclohexylring ist, an den gegebenenfalls ein weiterer Cycloalkylrest mit 5 bis 7 Ringkohlenstoffatomen annelliert ist.
- [G]: bedeutet in obiger Formel ein Strukturelement ausgewählt aus einer Einfachbindung und den Gruppen der Formel 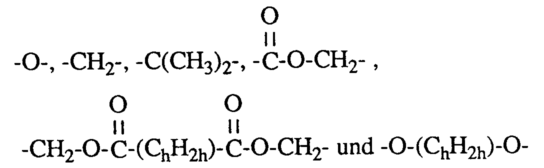 und
- h: eine ganze Zahl von 1 bis 6, insbesondere von 2 bis 4.

Spezielle Beispiele der zuletzt genannten Verbindungen sind die Verbindungen mit der Formel 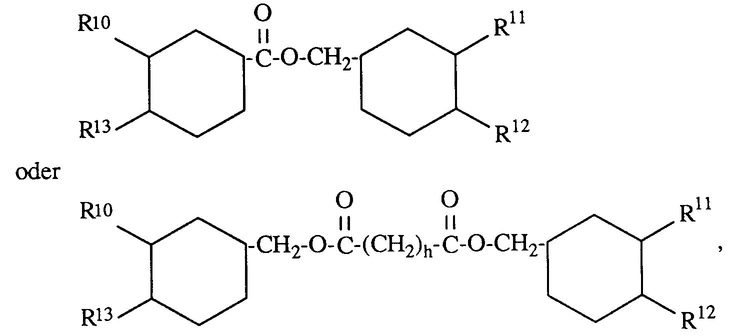 worin einer der Reste R¹⁰ oder R¹³ eine Gruppe der Formel H₂C=CH-O-(CH₂)ₓ-O- und der andere eine Gruppe der Formel R²O- und ebenso einer der Reste R¹¹ oder R¹² eine Gruppe der Formel H₂C=CH-O-(CH₂)ₓ-O- und der andere eine Gruppe der Formel R²O-darstellen, wobei x und h unabhängig voneinander jeweils eine ganze Zahl von 2 bis 4 bedeuten.

Die Vinyletherverbindungen, bei denen A eine Gruppe der Formel (2) darstellt, können beispielsweise analog zu dem oben beschriebenen Verfahren zur Herstellung von Vinyletherverbindungen der Formel (1) aus cycloaliphatischen Epoxidverbindungen der allgemeinen Strukturformel 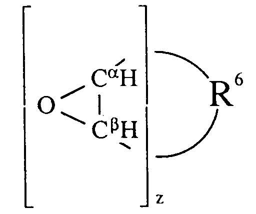 hergestellt werden, worin R⁶ einer organischen Gruppe in der oben schon definierten Bedeutung entspricht, indem man diese mit einem geeigneten Hydroxyalkylvinylether umsetzt und die sich bei dieser Reaktion bildende freie Hydroxylgruppe entweder in üblicher Weise in eine Glycidylethergruppe überführt oder mit dem gewünschten Diisocyanat R⁴(NCO)₂ und dem Alkohol R⁵OH umsetzt. Typische Beispiele für geeignete cycloaliphatische Epoxidverbindungen sind: 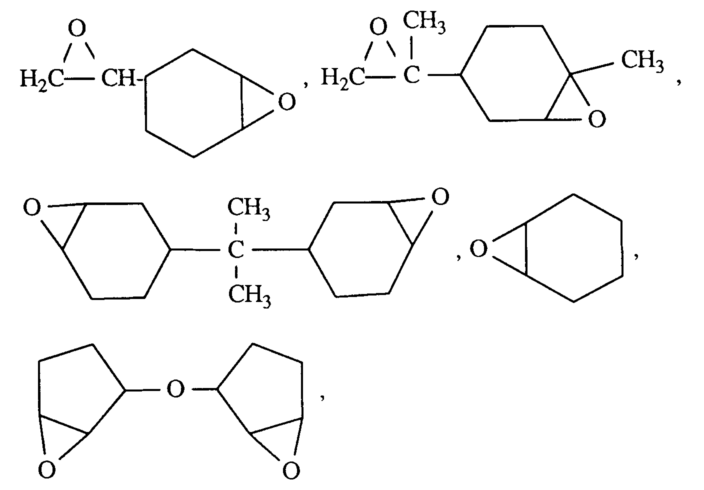 insbesondere dessen flüssiges Isomeres,

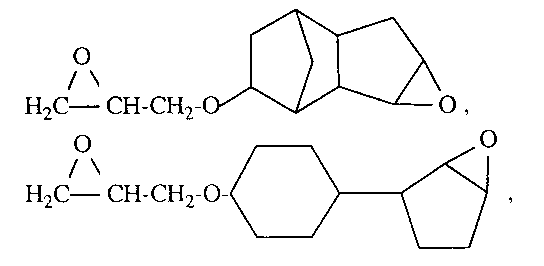

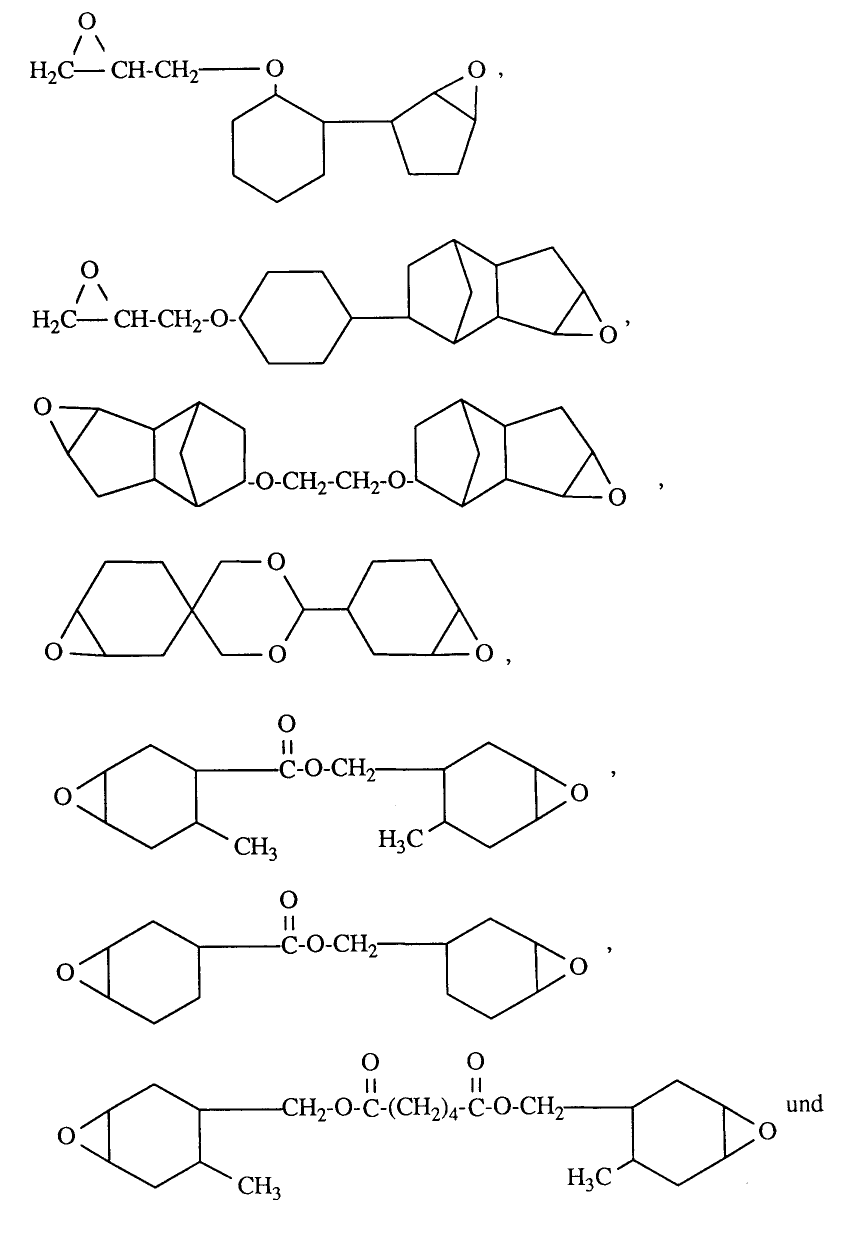

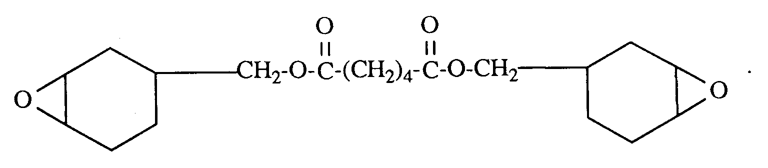

Von den Verbindungen der Formel [H₂C=CH-O-]_{z}A, bei denen
A einen Rest der Formel (3) oder (4) darstellt, werden im allgemeinen diejenigen bevorzugt, bei denen die Indices
- s: eine ganze Zahl von 2 bis 4, bevorzugt 2 oder 4,
- t: eine ganze Zahl von 0 bis 2,
- u: die Zahl 1,
- v: die Zahl 0 oder 1, und
- x: sowie
- y: eine ganze Zahl von 2 bis 10 bedeuten.

Hervorgehoben werden sollen weiterhin diejenigen Verbindungen des genannten Typs mit der Formel 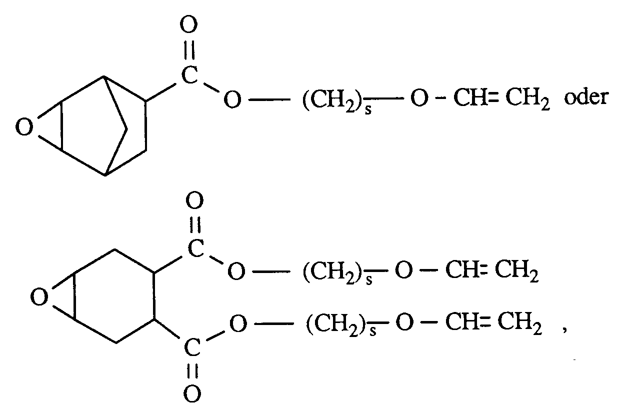 worin
- s: eine ganze Zahl von 2 bis 10, insbesondere 2 oder 4 beträgt.

Bei einer weiteren bevorzugten Ausführungsform dieser Vinyletherverbindungen, bei denen A einen Rest der Formel (4) darstellt, sind
- R¹⁴ und R¹⁵: beide ein Wasserstoffatom und
- z: ist gleich 1.

Herstellungsverfahren für die genannten Verbindungen sind dem Fachmannn bekannt.

So können die Verbindungen der Formel [H₂C=CH-O-]_{z}A, wo A einen Rest der Formel (3) darstellt, z. B. ausgehend von Carbonsäuren der Formeln HOOC-[D¹]-CH₃ oder HOOC-[D¹]-COOH, worin [D¹] einen Rest der Formel -(CₓH₂ₓ)-CH=CH-[-(CᵤH₂ᵤ])-CH=CH-]ᵥ-(C_{y}H_{2y})- darstellt und u, v ,x und y ebenfalls die oben schon genannte Bedeutung haben, oder ausgehend von entsprechenden Fettsäureestern, z. B. entsprechenden Methylestern, hergestellt werden, indem man diese zunächst in bekannter Weise mit in situ dargestellten Persäuren an den Doppelbindungen oxidiert und so in die entsprechenden Epoxyverbindungen überführt, welche danach mit dem gewünschten Hydroxyalkylvinylether der Formel H₂C=CH-O-(CₛH₂ₛ)-OH zum Endprodukt der Formel H₂C=CH-O-(CₛH₂ₛ)-OOC-[D]-CH₃ bzw. der Formel H₂C=CH-O-(CₛH₂ₛ)-OOC-[D]-COO-(CₛH₂ₛ)-O-CH=CH₂ umgesetzt werden.

Analog kann man zur Herstellung von Vinyletherverbindungen der Formel 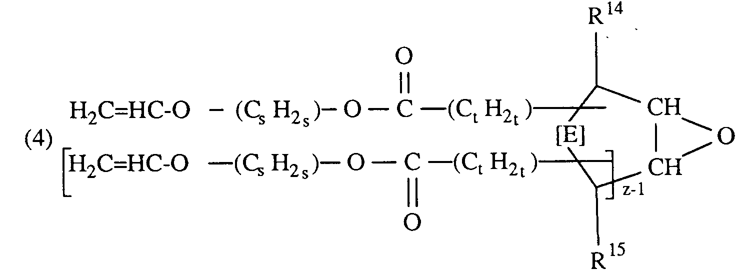 von den Verbindungen der Formel 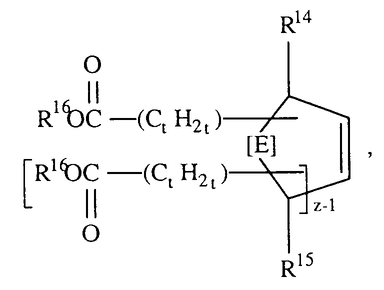 worin R¹⁶ ein Wasserstoffatom oder einer Alkylgruppe, z. B. eine Methylgruppe darstellt und die übrigen Symbole die oben schon definierte Bedeutung haben, ausgehen und diese nach einer analogen Epoxidierung ebenfalls mit den gewünschten Vinylethern der Formel H₂C=CH-O-(CₛH₂ₛ)-OH umsetzen.

Die erfindungsgemässen Vinyletherverbindungen stellen eine wertvolle Formulierungskomponente für strahlungsinduziert härtbare Zusammensetzungen dar. Die Erfindung betrifft daher auch deren Verwendung als eine polymerisierbare Komponente solcher Zusammensetzungen.

Derartige Zusammensetzungen enthalten weiterhin einen oder mehrere der weiter unten beschriebenen Photoinitiatoren in einer wirksamen Menge, z. B. von 0,5 bis 20 Gewichtsprozent, bezogen auf das Gesamtgewicht der Zusammensetzung.

Bevorzugt sind Zusammensetzungen, die neben einer oder mehreren der erfindungsgemässen Vinyletherverbindungen und einem oder mehreren Photoinitiatoren noch mindestens eine von den erfindungsgemässen Vinyletherverbindungen verschiedene polymerisierbare Verbindung enthalten.

Als zusätzliche polymerisierbare Verbindungen können beispielsweise übliche radikalisch polymerisierbare Verbindungen, im allgemeinen in Mengen von 0 bis 80 Gewichtsprozent, bezogen auf die gesamte Zusammensetzung, verwendet werden, wie Monoacrylate, Di- und Polyacrylate mit einer Acrylatfunktionalität bis zu 9 oder entsprechenden Methacrylate sowie Vinyl-Verbindungen mit einer Vinylfunktionalität bis zu 6.

Als Mono(meth-)acrylate eignen sich beispielsweise Allylacrylat, Allylmethacrylat, Methyl-, Ethyl-, n-Propyl-, n-Butyl-, Isobutyl-, n-Hexyl-, 2-Ethylhexyl-, n-Octyl-, n-Decyl- und n-Dodecylacrylat und -methacrylat, 2-Hydroxyethyl-, 2- und 3-Hydroxypropylacrylat und -methacrylat, 2-Methoxyethyl-, 2-Ethoxyethyl- und 2- oder 3-Ethoxypropylacrylat, Tetrahydrofurfurylmethacrylat, 2-(2-Ethoxyethoxy)ethylacrylat, Cyclohexylmethacrylat, 2-Phenoxyethylacrylat, Glycidylacrylat und Isodecylacrylat, sowie als Mono-N-vinylverbindung N-Vinylpyrrolidon oder N-Vinylcaprolactam. Solche Produkte sind ebenfalls bekannt und zum Teil im Handel erhältlich, zum Beispiel von der Firma SARTOMER Company.

Als zusätzliche Di(meth-)acrylate eignen sich beispielsweise die Di(meth-)acrylate von cycloaliphatischen oder aromatischen Diolen, wie 1 ,4-Dihydroxymethylcyclohexan, 2,2-Bis-(4-hydroxycyclohexyl)-propan, Bis-(4-hydroxycyclohexyl)-methan, Hydrochinon, 4,4'-Dihydroxybiphenyl, Bisphenol A, Bisphenol F, Bisphenol S, ethoxyliertes oder propoxyliertes Bisphenol A, ethoxyliertes oder propoxyliertes Bisphenol F oder ethoxyliertes oder propoxyliertes Bisphenol S. Solche Di(meth-)acrylate sind bekannt und zum Teil im Handel erhältlich.

Als Di(meth-)acrylate können auch Verbindungen der Formeln (4), (5), (6) oder (7) 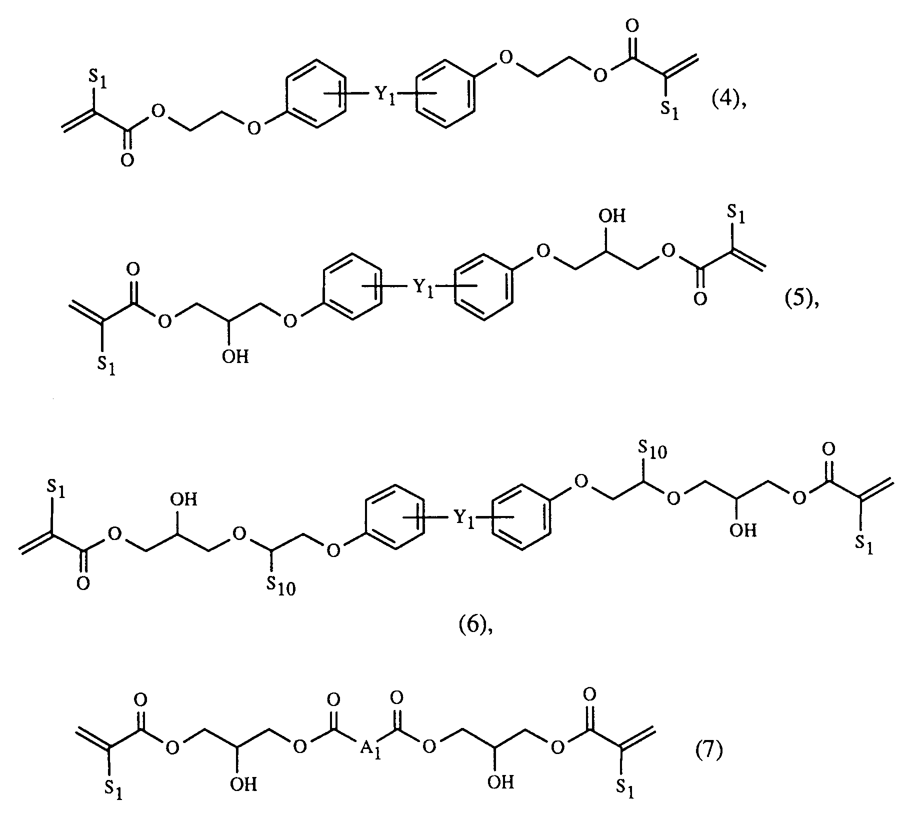 eingesetzt werden, worin
- S₁: ein Wasserstoffatom oder Methyl bedeutet,
- Y₁: für eine direkte Bindung, C₁-C₆-Alkylen, -S-, -O-, -SO-, -SO₂- oder -CO- steht,
- S₁₀: für eine C₁-C₈-Alkylgruppe, eine unsubstituierte oder durch eine oder mehrere C₁-C₄-Alkylgruppen, Hydroxygruppen oder Halogenatome substituierte Phenylgruppe, oder für einen Rest der Formel -CH₂-OS₁₁ steht, worin
- S₁₁: eine C₁-C₈-Alkylgruppe oder Phenylgruppe bedeutet und
- A₁: einen Rest aus gewählt aus den Resten der Formeln 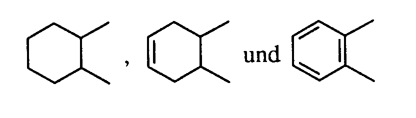 darstellt.

Die Di(meth-)acrylate der Formeln (4) und (5) sind bekannt und zum Teil im Handel erhältlich, beispielsweise unter der Bezeichung SR®349 oder Novacure®3700, und können hergestellt werden, indem man ethoxylierte Bisphenole, insbesondere ethoxyliertes Bisphenol A, oder Bisphenoldiglycidylether, insbesondere Bisphenol A-diglycidylether, mit (Meth)acrylsäure zu den Verbindungen der Formeln (4) oder (5) umsetzt.

In gleicher Weise können auch die Verbindungen der Formeln (6) und (7) hergestellt werden, indem man einen Diglycidylether der Formel (6a) 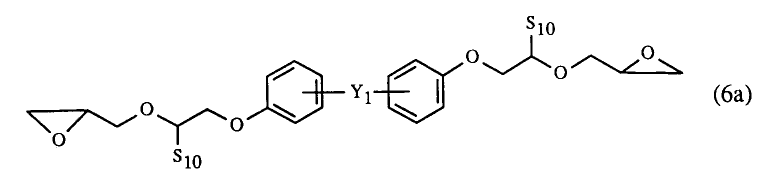 oder einen Diglycidylester der Formel (7a) 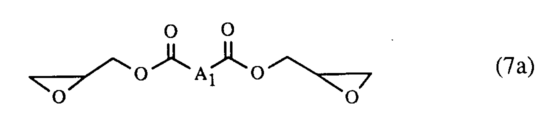 mit (Meth)acrylsäure zu den Verbindungen der Formel (6) oder (7) umsetzt, wobei S₁₀, Y₁ und A₁ die oben angegebene Bedeutung haben.

Ferner können als Diacrylate auch eine Verbindung der Formeln (8), (9), (10) oder (11) 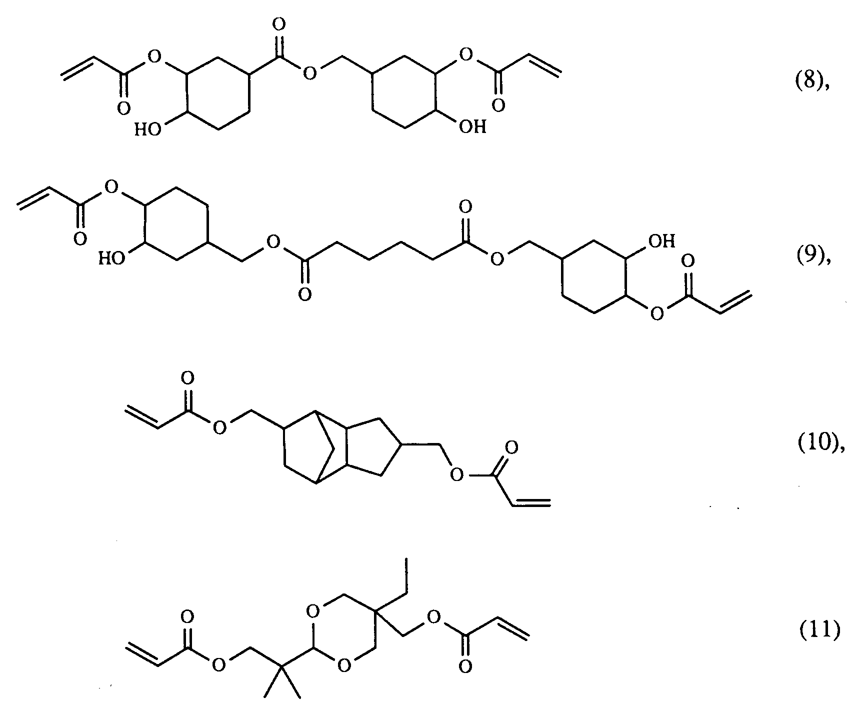 verwendet werden. Diese Verbindungen sind bekannt und zum Teil im Handel erhältlich. Beispielsweise können die Verbindungen der Formeln (8) und (9) in bekannter Weise durch Umsetzen der cycloaliphatischen Diepoxide der Formeln (8a) beziehungsweise (9a) 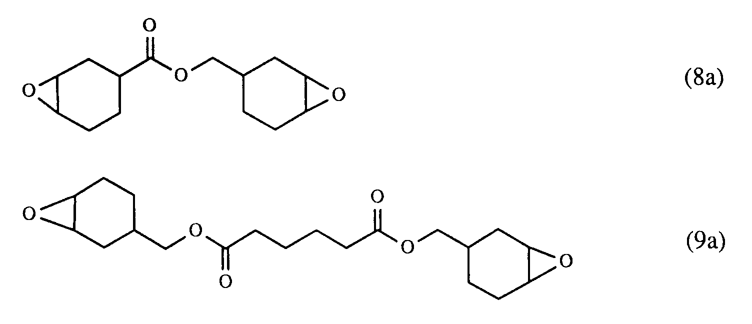 mit (Meth)acrylsäure zu den Verbindungen der Formeln (8) oder (9) erhalten werden. Die Verbindung der Formel (11) ist im Handel unter der Bezeichung Kayarad®R-604 erhältlich.

Als zusätzliche Poly(meth-)acrylate kommen z. B. monomere oder oligomere aliphatische, cycloaliphatische oder aromatische Acrylate oder Methacrylate mit einer (Meth-)Acrylatfunktionalität von grösser als 2, insbesondere tri-, tetra- oder pentafunktionelle Acrylate oder Methacrylate, in Frage.

Als aliphatische polyfunktionelle (Meth-)Acrylate eignen sich beispielsweise die Triacrylate und Trimethacrylate von Hexan-2,4,6-triol, Glycerin oder 1,1,1-Trimethylolpropan, ethoxyliertes oder propoxyliertes Glycerin oder 1,1,1-Trimethylolpropan und die hydroxygruppenhaltigen Tri(meth-)acrylate, die durch Umsetzung von Triepoxidverbindungen, wie beispielsweise die Triglycidylether von den genannten Triolen, mit (Meth-)Acrylsäure erhalten werden. Weiterhin können zum Beispiel Pentaerythritoltetraacrylat, Bistrimethylolpropantetraacrylat, Pentaerythritolmonohydroxytriacrylat oder -methacrylat oder Dipentaerythritolmonohydroxypentaacrylat oder -methacrylat verwendet werden.

Ausserdem können als weitere strahlungsinduziert polymerisierbare Verbindungen in den erfindungsgemässen Zusammensetzungen hexafunktionelle oder höherfunktionelle Urethanacrylate oder Urethanmethacrylate verwendet werden. Diese Urethan(meth-)acrylate sind dem Fachmann bekannt und können in bekannter Weise hergestellt werden, indem man beispielsweise ein hydroxylterminiertes Polyurethan mit Acrylsäure oder Methacrylsäure umsetzt, oder indem man ein isocyanatterminiertes Präpolymer mit Hydroxyalkyl(meth)acrylaten zum Urethan(meth-)acrylat umsetzt.

Als aromatische Tri(meth-)acrylate eignen sich beispielsweise die Umsetzungsprodukte aus Triglycidylethern dreiwertiger Phenole und drei Hydroxylgruppen aufweisende Phenol- oder Kresolnovolake mit (Meth)-Acrylsäure.

Vorzugsweise enthalten die erfindungsgemässen Zusammensetzungen mindestens ein flüssiges, (Meth)acrylat mit einer Acrylatfunktionalität von 1 bis 9, besonders bevorzugt enthalten sie eine flüssige Mischung aus aromatischen, aliphatischen oder cycloaliphatischen (Meth)acrylaten mit einer Acrylatfunktionalität von 1 bis 9.

Weiterhin können die erfindungsgemässen Zusammensetzungen als zusätzliche polymerisierbare Komponenten kationisch polymerisierbares organisches Material enthalten, im allgemeinen ebenfalls in Mengen von 0 bis 80 Gewichtsprozent, bezogen auf die gesamte Zusammensetzung. Hierbei handelt es sich insbesondere um Epoxidverbindungen, bevorzugt solche, die bei Temperaturen von ungefähr 10 bis 30 °C fliessfähig sind. Bei den Epoxidharzen, die in den erfindungsgemässen Zusammensetzungen Verwendung finden, handelt es sich im allgemeinen um Verbindungen, die im Durchschnitt mehr als eine 1,2-Epoxidgruppen im Molekül besitzen.

Solche Harze können eine aliphatische, aromatische, cycloaliphatische, araliphatische oder heterocyclische Struktur haben; sie enthalten Epoxidgruppen als Seitengruppen, oder diese Gruppen bilden einen Teil eines alicyclischen oder heterocyclischen Ringsystems. Epoxyharze dieser Typen sind allgemein bekannt und im Handel erhältlich.

Beispielhaft für Epoxyharze dieses Typs sind zu erwähnen:
I) Polyglycidyl- und Poly-(β-methylglycidyl)-ester erhältlich durch Umsetzung einer Verbindung mit mindestens zwei Carboxylgruppen im Molekül und Epichlorhydrin bzw. Glycerindichlorhydrin bzw. β-Methyl-epichlorhydrin. Die Umsetzung erfolgt zweckmässig in Gegenwart von Basen.
   Als Verbindungen mit mindestens zwei Carboxylgruppen im Molekül können aliphatische Polycarbonsäuren verwendet werden. Beispiele für diese Polycarbonsäuren sind Glutarsäure, Adipinsäure, Pimelinsäure, Korksäure, Azelainsäure, Sebazinsäure oder dimerisierte bzw. trimerisierte Linolsäure.
   Es können aber auch cycloaliphatische Polycarbonsäuren eingesetzt werden, wie beispielsweise Tetrahydrophthalsäure, 4-Methyltetrahydrophthalsäure, Hexahydrophthalsäure oder 4-Methylhexahydrophthalsäure.
   Weiterhin können aromatische Polycarbonsäuren Verwendung finden, wie beispielsweise Phthalsäure, Isophthalsäure, Trimellitsäure oder Pyromellitsäure.
   Ebenfalls können auch carboxylterminierte Addukte, z.B. von Trimellitsäure und Poly-olen, wie beispielsweise Glycerin oder 2,2-Bis-(4-hydroxycyclohexyl)-propan, verwendet werden.
II) Polyglycidyl- oder Poly-(β-methylglycidyl)-ether erhältlich durch Umsetzung einer Verbindung mit mindestens zwei freien alkoholischen Hydroxygruppen und/oder phenolischen Hydroxygruppen und einem geeignet substituierten Epichlorhydrin unter alkalischen Bedingungen, oder in Anwesenheit eines sauren Katalysators und anschliessender Alkalibehandlung.
   Ether dieses Typs leiten sich beispielsweise ab von acyclischen Alkoholen, wie Ethylenglykol, Diethylenglykol und höheren Poly-(oxyethylen)-glykolen, Propan-1,2-diol, oder Poly-(oxypropylen)-glykolen, Propan-1,3-diol, Butan-1,4-diol, Poly-(oxytetramethylen)-glykolen, Pentan-1,5-diol, Hexan-1,6-diol, Hexan-2,4,6-triol, Glycerin, 1,1,1-Trimethylolpropan, Bistrimethylolpropan, Pentaerythrit, Sorbit, sowie von Polyepichlorhydrinen.
   Sie leiten sich aber auch beispielsweise ab von cycloaliphatischen Alkoholen, wie 1,3-oder 1,4-Dihydroxycyclohexan, Bis-(4-hydroxycyclohexyl)-methan, 2,2-Bis-(4-hydroxycyclohexyl)-propan oder 1,1-Bis-(hydroxymethyl)-cyclohex-3-en, oder sie besitzen aromatische Kerne, wie N,N-Bis-(2-hydroxyethyl)-anilin oder p,p'-Bis-(2-hydroxyethylamino)-diphenylmethan.
   Die Epoxidverbindungen können sich auch von einkernigen Phenolen ableiten, wie beispielsweise von Resorcin oder Hydrochinon, oder sie basieren auf mehrkernigen Phenolen, wie beispielsweise auf Bis-(4-hydroxyphenyl)-methan (Bisphenol F), 2,2-Bis-(4-hydroxyphenyl)-propan (Bisphenol A), oder auf unter sauren Bedingungen erhaltenen Kondensationsprodukten von Phenolen oder Kresolen mit Formaldehyd, wie Phenol-Novolake und Kresol-Novolake.
III) Poly-(N-glycidyl)-Verbindungen sind beispielsweise erhältlich durch Dehydrochlorierung der Reaktionsprodukte von Epichlorhydrin mit Aminen, die mindestens zwei Aminwasserstoffatome enthalten. Bei diesen Aminen handelt es sich zum Beispiel um n-Butylamin, Anilin, Toluidin, m-Xylylendiamin, Bis-(4-aminophenyl)-methan oder Bis-(4-methylaminophenyl)-methan.
   Zu den Poly-(N-glycidyl)-Verbindungen zählen aber auch N,N'-Diglycidylderivate von Cycloalkylenharnstoffen, wie Ethylenharnstoff oder 1,3-Propylenharnstoff, und N,N'-Diglycidylderivate von Hydantoinen, wie von 5,5-Dimethylhydantoin.
IV) Beispiele für Poly-(S-glycidyl)-Verbindungen sind Di-S-glycidylderivate, die sich von Dithiolen, wie beispielsweise Ethan-1,2-dithiol oder Bis-(4-mercaptomethylphenyl)-ether, ableiten.
V) Beispiele für Epoxidverbindungen, worin die Epoxidgruppen einen Teil eines alicyclischen oder heterocyclischen Ringsystems bilden, sind beispielsweise Bis-(2,3-epoxicyclopentyl)-ether, 2,3-Epoxicyclopentylglycidylether, 1,2-Bis-(2,3-epoxicyclopentyloxy)-ethan, Bis-(4-hydroxycyclohexyl)-methandiglycidylether, 2,2-Bis-(4-hydroxycyclohexyl)-propandiglycidylether, 3,4-Epoxycyclohexylmethyl-3,4-epoxycyclohexancarboxylat, 3,4-Epoxy-6-methyl-cyclohexylmethyl-3,4-epoxy-6-methylcyclohexancarboxylat, Di-(3,4-epoxycyclohexylmethyl)hexandioat, Di-(3,4-epoxy-6-methyl-cyclohexylmethyl)-hexandioat, Ethylenbis-(3,4-epoxycyclohexancarboxylat), Ethandioldi-(3,4-epoxycyclohexylmethyl)-ether, Vinylcyclohexendioxid, Dicyclopentadiendiepoxid oder 2-(3,4-Epoxycyclohexyl-5,5-spiro-3,4-epoxy)cyclohexan-1,3-dioxan.

Es lassen sich aber auch Epoxidharze verwenden, bei denen die 1,2-Epoxidgruppen an unterschiedliche Heteroatome bzw. funktionelle Gruppen gebunden sind. Zu diesen Verbindungen zählen beispielsweise das N,N,O-Triglycidylderivat des 4-Aminophenols, der Glycidylether-glycidylester der Salicylsäure, N-Glycidyl-N'-(2-glycidyloxypropyl)-5,5-dimethylhydantoin oder 2-Glycidyloxy-1,3-bis-(5,5-dimethyl-1-glycidylhydantoin-3-yl)-propan.

Ferner sind auch flüssige vorreagierte Addukte solcher Epoxyharze mit Härtern für Epoxidharze geeignet.

Natürlich können auch Mischungen von Epoxidharzen in den erfindungsgemässen Zusammensetzungen verwendet werden.

Als Photoinitiatoren können, abhängig von den polymerisierbaren Gruppen, die die Komponenten der erfindungsgemässen Zusammensetzungen aufweisen, sowohl radikalische Photoinitiatoren als auch Photoinitatoren für die kationische Polymerisation sowie Gemische eines oder mehrerer der genannten Initiatoren eingesetzt werden.

Radikalische Photoinitiatoren kommen insbesondere bei Anwesenheit von Verbindungen mit Kohlenstoff-Kohlenstoff-Doppelbindungen in den erfindungsgemässen Zusammensetzungen zur Anwendung, insbesondere bei Anwesenheit von Verbindungen mit Acrylat- , Methacrylat- und Vinylgruppen. Im allgemeinen können hierbei alle Typen von Photoinitiatoren eingesetzt werden, welche bei der entsprechenden Bestrahlung freie Radikale bilden. Typische Verbindungen bekannter Photoinitiatoren sind Benzoine, wie Benzoin, Benzoinether, wie Benzoinmethylether, Benzoinethylether und Benzoinisopropylether, Benzoinphenylether und Benzoinacetat, Acetophenone, wie Acetophenon, 2,2-Dimethoxyacetophenon und 1,1-Dichloracetophenon, Benzil, Benzilketale, wie Benzildimethylketal und Benzildiethylketal, Anthrachinone, wie 2-Methylanthrachinon, 2-Ethylanthrachinon, 2-tert-Butylanthrachinon, 1-Chloranthrachinon und 2-Amylanthrachinon, ferner Triphenylphosphin, Benzoylphosphinoxide, wie beispielsweise 2,4,6-Trimethylbenzoyldiphenylphosphinoxid (Luzirin TPO), Benzophenone, wie Benzophenon und 4,4'-Bis-(N,N'-dimethylamino)-benzophenon, Thioxanthone und Xanthone, Acridinderivate, Phenazinderivate, Quinoxalinderivate oder 1-Phenyl-1,2-propandion-2-O-benzoyloxim, 1-Aminophenylketone oder 1-Hydroxyphenylketone, wie 1-Hydroxycyclohexylphenylketon, Phenyl-(1-hydroxyisopropyl)-keton und 4-Isopropylphenyl-(1-hydroxyisopropyl)-keton, welche alle bekannte Verbindungen darstellen.

Besonders geeignete Photoinitiatoren, welche gewöhnlich in Kombination mit einem He/Cd-Laser als Lichtquelle verwendet werden, sind Acetophenone, wie 2,2-Dialkoxybenzophenone und 1 -Hydroxyphenylketone, beispielsweise 1-Hydroxycyclohexylphenylketon oder 2-Hydroxyisopropylphenylketon (= 2-Hydroxy-2,2-dimethylacetophenon), insbesondere aber 1-Hydroxycyclohexylphenylketon.

Eine andere Klasse von radikalischen Photoinitiatoren, die gewöhnlich bei Verwendung von Argonion-Lasern eingesetzt wird, sind die Benzilketale, wie beispielsweise Benzildimethylketal. Insbesondere verwendet man als Photoinitiator ein α-Hydroxyphenylketon, Benzildimethylketal oder 2,4,6-Trimethylbenzoyldiphenylphosphinoxid.

Eine weitere Klasse von geeigneten radikalischen Photoinitiatoren stellen die ionischen Farbstoff-Gegenionverbindungen (ionic dye-counter ion compounds) dar, welche in der Lage sind, aktinische Strahlen zu absorbieren und freie Radikale zu erzeugen, die die Polymerisation von Substanzen, wie (Meth)acrylaten oder Vinylverbindungen initiieren. Die erfindungsgemässen Gemische, welche ionische Farbstoff-Gegenionverbindungen enthalten, können auf diese Weise mit sichtbarem Licht im einstellbaren Wellenlängenbereich von 400-700 nm variabler gehärtet werden. Ionische Farbstoff-Gegenionverbindungen und deren Wirkungsweise sind bekannt, beispielsweise aus der EP-A-O 223 587 und den US-Patenten 4,751,102; 4,772,530 und 4,772,541. Als Beispiele für geeignete ionische Farbstoff-Gegenionverbindungen seien genannt die anionischen Farbstoff-Jodoniumionkomplexe, die anionischen Farbstoff-Pyrylliumionkomplexe und insbesondere die kationischen Farbstoff-Boratanionverbindungen der Formel 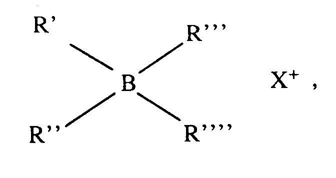 worin X⁺ für einen kationischen Farbstoff steht und R', R'', R''' und R'''' unabhängig voneinander je ein Alkyl, Aryl, Alkaryl, Allyl, Aralkyl, Alkenyl, Alkinyl, eine alicyclische oder gesättigte oder ungesättigte heterocyclische Gruppe bedeuten.

Als Photoinitiatoren für Komponenten mit kationisch polymerisierbaren Gruppen, insbesondere Epoxid- oder Vinylethergruppen, können praktisch alle in der Technik hierfür bekannten Verbindungen eingesetzt werden. Hierzu zählen beispielsweise Oniumsalze mit Anionen schwacher Nukleophilie. Beispiele dafür sind Haloniumsalze, Iodosylsalze oder Sulfoniumsalze, wie sie in der EP-A 153 904 beschrieben sind, Sulfoxoniumsalze, wie beispielsweise in den EP-A 35 969, 44 274, 54 509 und 164 314 beschrieben oder Diazoniumsalze, wie beispielsweise in der US-A 3,708,296 beschrieben. Weitere kationische Photoinitiatoren sind Metallocensalze, wie bespielsweise in den EP-A 94 914 und 94 915 beschrieben.

Eine Übersicht über weitere gängige Oniumsalzinitiatoren und/oder Metallocensalze bieten "UV-Curing, Science and Technology", (Editor: S.P. Pappas, Technology Marketing Corp., 642 Westover Road, Stanford, Conneticut, USA) oder "Chemistry & Technology of UV & EB Formulation for Coatings, Inks & Paints", Vol. 3 (edited by P. K. T. Oldring).

Bevorzugt als Photoinitiatoren für kationisch polymerisierbare Gruppen sind Verbindung der Formeln (12), (13) oder (14) 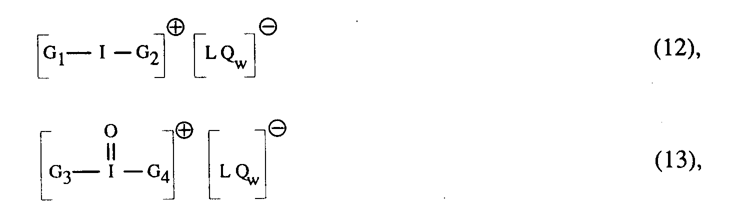 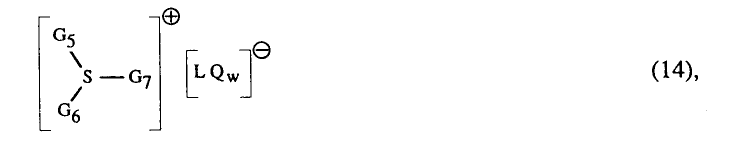 worin G₁, G₂, G₃, G₄, G₅, G₆ und G₇ unabhängig voneinander unsubstituiertes oder durch geeignete Reste substituiertes C₆-C₁₈-Aryl bedeuten,
- L: Bor, Phosphor, Arsen oder Antimon darstellt,
- Q: ein Halogenatom ist oder ein Teil der Reste Q in einem Anion LQ_{w}⁻ auch Hydroxylgruppen sein kann und
- w: eine ganze Zahl ist, die der um 1 vergrösserten Wertigkeit von L entspricht.

Beispiele für C₆-C₁₈-Aryl sind Phenyl, Naphthyl, Anthryl und Phenanthryl. Gegebenenfalls vorliegende Substituenten für geeignete Reste sind Alkyl, bevorzugt C₁-C₆-Alkyl, wie Methyl, Ethyl, n-Propyl, Isopropyl, n-Butyl, sec-Butyl, Isobutyl, tert-Butyl oder die verschiedenen Pentyl- oder Hexyl-Isomere, Alkoxy, bevorzugt C₁-C₆-Alkoxy, wie Methoxy, Ethoxy, Propoxy, Butoxy, Pentoxy oder Hexoxy, Alkylthio, bevorzugt C₁-C₆-Alkylthio, wie Methylthio, Ethylthio, Propylthio, Butylthio, Pentylthio oder Hexylthio, Halogen, wie Fluor, Chlor, Brom oder Jod, Aminogruppen, Cyanogruppen, Nitrogruppen oder Arylthio, wie Phenylthio.

Beispiele für bevorzugte Halogenatome Q sind Chlor und insbesondere Fluor. Bevorzugte Anionen LQₘ⁻ sind BF₄⁻, PF₆⁻, AsF₆⁻, SbF₆⁻ und SbF₅(OH)⁻. Weiterhin stellt CF₃SO₃⁻ ein bevorzugtes Kation dar.

Selbstverständlich sind auch solche Verbindungen als Initiatoren geeignet, die zwei oder mehr der Oniumgruppen im Molekül enthalten, beispielsweise Disulfoniumverbindungen.

Besonders bevorzugt sind kationische Photoinitiatoren der Formel (14), worin G₅, G₆ und G₇ Phenyl oder Biphenyl bedeutet oder Gemische dieser beiden Verbindungen.

Ein weiterer bevorzugter Typ kationische Photoinitatoren hat die Formel (15) 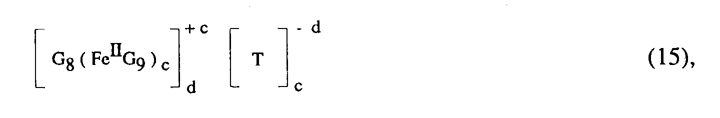 worin
- c: 1 oder 2 bedeutet,
- d: 1, 2, 3, 4 oder 5 ist,
- T: für ein nicht nukleophiles Anion, z. B. BF₄⁻, PF₆⁻, AsF₆⁻, SbF₆⁻, CF₃SO₃⁻, C₂F₅SO₃⁻, n-C₃F₇SO₃⁻, n-C₄F₉SO₃⁻, n-C₆F₁₃SO₃⁻, n-C₈F₁₇SO₃⁻, C₆F₅SO₃⁻, Phosphorwolframat (PO₄₀W₁₂³⁻) oder Siliciumwolframat (SiO₄₀W₁₂⁴⁻) steht,
- G₈: ein π-Aren ist und
- G₉: ein Anion eines π-Arens, insbesondere ein Cyclopentadienylanion, bedeutet.

Beispiele für π-Arene als G₈ und Anionen von π-Arenen G₉ als findet man in der EP-A 94 915. Beispiele für bevorzugte π-Arene als G₈ sind Toluol, Xylol, Ethylbenzol, Cumol, Methoxybenzol, Methylnaphthalin, Pyren, Perylen, Stilben, Diphenylenoxid und Diphenylensulfid. Insbesondere bevorzugt sind Cumol, Methylnaphthalin oder Stilben.

Als Anion T werden PF₆⁻, AsF₆⁻, SbF₆⁻, CF₃SO₃⁻, C₂F₅SO₃⁻, n-C₃F₇SO₃⁻, n-C₄F₉SO₃⁻, n-C₆F₁₃SO₃⁻ und n-C₈F₁₇SO₃⁻ bevorzugt.

Die Metallocensalze können auch in Kombination mit Oxidationsmitteln eingesetzt werden. Solche Kombinationen sind in der EP-A 126 712 beschrieben.

Zur Erhöhung der Lichtausbeute können je nach Initiatortyp auch Sensibilisatoren eingesetzt werden. Beispiele hierfür sind polycyclische aromatische Kohlenwasserstoffe oder aromatische Ketoverbindungen. Spezifische Beispiele bevorzugter Sensibilisatoren sind in der EP-A 153 904 erwähnt.

Photoinitiatoren werden in wirksamen Mengen zugesetzt, beispielsweise jeweils in Mengen von etwa 0,1 bis etwa 10 Gewichtsprozent, bezogen auf die Gesamtmenge des Gemisches. Wenn die erfindungsgemässen Gemische für stereolithographische Verfahren verwendet werden, wobei normalerweise Laserstrahlen eingesetzt werden, ist es wesentlich, dass die Absorptionsfähigkeit der Gemische durch Typ und Konzentration des Photoinitiators so abgestimmt wird, dass die Härtungstiefe bei normaler Lasergeschwindigkeit ungefähr 0,1 bis 2,5 mm beträgt. Bevorzugt liegt die Gesamtmenge an Photoinitiatoren in den erfindungsgemässen Zusammensetzungen zwischen 0,5 und 5 Gewichtsprozent.

Die erfindungsgemässen Gemische können auch verschiedene Photoinitiatoren enthalten, welche gegenüber Strahlen von Emissionslinien unterschiedlicher Wellenlängen verschieden strahlungsempfindlich sind. Man erreicht dadurch beispielsweise eine bessere Ausnützung einer UV/VIS-Lichtquelle, welche Emissionslinien unterschiedlicher Wellenlänge ausstrahlt. Vorteilhaft ist es dabei, wenn die verschiedenen Photoinitiatoren so ausgewählt und in einer solchen Konzentration eingesetzt werden, dass bei den verwendeten Emissionslinien eine gleiche optische Absorption erzeugt wird.

Oft ist es zweckmässig, den erfindungsgemässen Zusammensetzungen weitere Bestandteile zuzsetzen, z. B. übliche Additive, wie Stabilisatoren, z. B. UV-Stabilisatoren, Polymerisationsinhibitoren, Trennmittel, Benetzungsmittel, Verlaufsmittel, Sensibilisatoren, Antiabsetzmittel, oberflächenaktive Mittel, Farbstoffe, Pigmente oder Füllstoffe. Diese werden jeweils in einer für den erwünschten Zweck wirksamen Menge eingesetzt und können insgesamt z. B. bis 20 Gewichtsprozent der erfindungsgemässen Zusammensetzungen ausmachen.

Die Zusammensetzungen können gegebenenfalls auch bis 50 Gewichtsprozent eines hydroxylterminierten Polyethers oder Polyesters enthalten, z. B. di- oder trifunktionelle Polyether oder Polyester-Polyole, Polytetrahydrofuran, hydroxylterminierte Polyurethane oder als bevorzugte der genannten Komponenten Poly-ε-caprolactam.

Bevorzugte Zusammensetzungen enthalten
- 5 bis 60 Gew.-%: einer oder mehrerer der erfindungsgemässen Vinyletherverbindungen;
- 0 bis 40 Gew.-%: mono-, di- oder mehrfunktionellen Acrylate oder Methacrylate;
- 30 bis 80 Gew.-%: di- oder polyfunktionelle Epoxidverbindungen;
- 0 bis 5 Gew.-%: radikalische Photoinitiatoren;
- 0,5 bis 5 Gew.-%: kationische Photoinitiatoren;
- 0 bis 40 Gew.-%: hydroxylterminierte Polyether oder Polyester; und
- 0 bis 10 Gew.-%: eines oder mehrerer Additive.

Die Zusammensetzungen können in bekannter Weise hergestellt werden, beispielsweise durch Vormischen einzelner Komponenten und anschliessendes Vermischen dieser Vormischungen oder durch Vermischen aller Komponenten mittels üblicher Vorrichtungen, wie Rührbehälter, in Abwesenheit von Licht und gegebenenfalls bei leicht erhöhter Temperatur.

Die erfindungsgemässen Zusammensetzungen können durch Bestrahlen mit aktinischem Licht, beispielsweise mittels Elektronen-, Röntgenstrahlen, UV- oder VIS-Licht, das heisst, vorzugsweise mit Strahlen im Wellenlängenbereich von 280-650 nm polymerisiert werden. Besonders geeignet sind Laserstrahlen von HeCd, Argon oder Stickstoff sowie Metalldampf und NdYAG-Laser. Es ist dem Fachmann bekannt, dass für jede gewählte Lichtquelle der geeignete Photoinitiator ausgewählt und gegebenenfalls sensibilisiert werden muss. Man hat erkannt, dass die Eindringtiefe der Strahlen in die zu polymerisierende Zusammensetzung und die Arbeitsgeschwindigkeit in direktem Zusammenhang mit dem Absorptionskoeffizienten und der Konzentration des Photoinitiators stehen. In der Stereolithographie werden vorzugsweise solche Photoinitiatoren eingesetzt, welche die höchste Anzahl von entstehenden freien Radikalen beziehungsweise kationischen Partikeln bewirken und die grösste Strahlungseindringtiefe in die zu polymerisierenden Zusammensetzungen ermöglichen.

Gegenstand der Erfindung ist auch ein Verfahren zur Herstellung eines gehärteten Produkts, bei dem man Zusammensetzungen, wie sie oben beschrieben sind, mit aktinischer Strahlung behandelt. Beispielsweise können die erfindungsgemässen Zusammensetzungen hierbei als Klebstoffe, als Beschichtungsmittel, als Photoresists, z. B. als Lötstoppresist, oder für das Rapid-Prototyping, insbesondere für die Stereolithographie verwendet werden.

Ein weiterer Erfindungsgegenstand ist daher ein Verfahren zur Herstellung von dreidimensionalen Gegenständen aus den flüssigen, erfindungsgemässen Gemischen mittels stereolithographischen Verfahren, umfassend einen Schritt, bei dem die Oberfläche einer Schicht aus dem erfindungsgemässen, flüssigen Gemisch ganzflächig oder in einem vorbestimmten Muster mit einer UV/VIS-Lichtquelle bestrahlt wird, so dass sich in den bestrahlten Bereichen eine Schicht in einer gewünschten Schichtdicke verfestigt, dann eine neue Schicht aus den erfindungsgemässen Gemischen auf der verfestigten Schicht gebildet wird, die ebenfalls ganzflächig oder in einem vorbestimmten Muster bestrahlt wird, und wobei durch wiederholtes Beschichten und Bestrahlen dreidimensionale Gegenstände aus mehreren aneinander haftenden, verfestigten Schichten erhalten werden.

Bei diesem Verfahren wird als Strahlungsquelle vorzugsweise ein Laserstrahl verwendet, der vorzugsweise computergesteuert ist.

Im allgemeinen erfolgt nach der oben beschriebenen ersten Strahlungshärtung, bei der die sogenannten Grünlinge erhalten werden, die noch keine ausreichende Festigkeit zeigen, die endgültige Aushärtung der Formkörper durch Erhitzen und/oder weiteres Bestrahlen.

Werden die erfindungsgemässen Gemische als Beschichtungsmittel eingesetzt, so erhält man klare und harte Beschichtungen auf Holz, Papier, Metall, Keramik oder anderen Oberflächen. Die Beschichtungsdicke kann dabei sehr variieren und etwa von 1 µm bis etwa 1 mm betragen. Aus den erfindungsgemässen Gemischen können Reliefbilder für gedruckte Schaltungen oder Druckplatten direkt durch Bestrahlen der Gemische hergestellt werden, beispielsweise mittels eines computergesteuerten Laserstrahls geeigneter Wellenlänge oder unter Anwendung einer Photomaske und einer entsprechenden Lichtquelle.

### Beispiel 1: Herstellung einer Vinyletherverbindung mit der Formel:

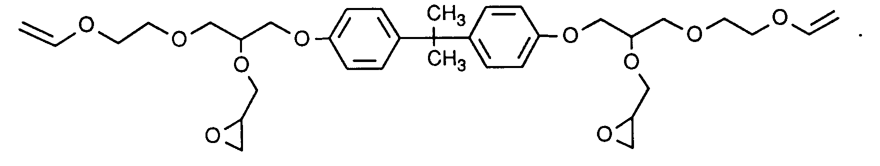

A) 111,7 g (0,3 Mol) Bisphenol-A-diglycidylether (Araldit®GY 250) werden mit 52,87 g (0,6 Mol) Hydroxyethylvinylether und 0,1 g KOH unter Stickstoffatmosphäre auf 120 °C erhitzt. Dann wird die Mischung 12 Stunden lang bei dieser Temperatur und danach 48 Stunden bei 150 °C gerührt.
B) 63 g (0,12 Mol) des so erhaltenenen Divinylethers werden mit 178 g (1,92 Mol) Epichlorhydrin und 1,57 g einer 50%-igen Tetramethylammoniumchloridlösung (TMAC) bei 70 °C eine Stunde lang gerührt. Dann werden unter reduziertem Druck 19,2 g einer 50%-igen NaOH-Lösung zugetropft (90 mbar; 79 °C), wobei das entstehende Wasser im Wasserabscheider abgetrennt wird. Nach beendeter Wasserabscheidung wird das überschüssige Epichlorhydrin abdestilliert und der Rückstand mit 200 Milliliter Toluol aufgenommen. Das entstandene NaCl wird abfiltriert und das Filtrat mit NaHCO₃-Lösung und Wasser extrahiert. Die organische Phase wird schliesslich getrocknet und das Lösungsmittel im Rotationsverdampfer entfernt. Es werden 70,8g (93,8% der theoretischen Ausbeute) des erwünschten Produktes in Form eines viskosen, bräunlichen Harzes erhalten, dessen Epoxidgehalt 2,8 Äquivalente/kg beträgt (88,3% der Theorie).

### Beispiel 2: Herstellung einer Vinyletherverbindung mit der Formel:

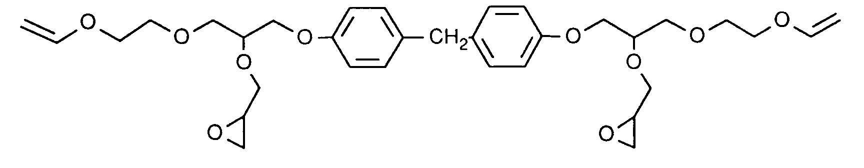

A) 156,2 g (0,5 Mol) Bisphenol-F-diglycidylether (Araldit®PY 306) werden mit 88,11 g (1,0 Mol) Hydroxyethylvinylether und 0,17 g KOH unter Stickstoffatmosphäre auf 120 °C erhitzt. Dann wird die Mischung 12 Stunden lang bei dieser Temperatur und danach 48 Stunden bei 150 °C gerührt.
B) 150 g (0,5 Mol) des so erhaltenenen Divinylethers werden mit 499,54 g (5,4 Mol) Epichlorhydrin und 4,5 g einer 50%-igen Tetramethylammoniumchloridlösung bei 70 °C eine Stunde lang gerührt. Dann werden unter reduziertem Druck 54,4 g einer 50%-igen NaOH-Lösung zugetropft, und es wird unter Wasserabscheidung gemäss Beispiel 1 umgesetzt und aufgearbeitet. Es werden 148 g (78% der theoretischen Ausbeute) des erwünschten Produktes in Form einer viskosen, gelblichen Flüssigkeit erhalten, dessen Epoxidgehalt 2,55 Äquivalente/kg beträgt (71,1% der Theorie).

### Beispiel 3: Herstellung einer Vinyletherverbindung mit der Formel

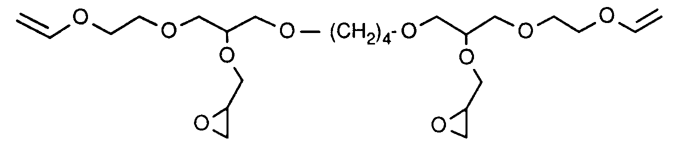

A) 101,13 g (0,5 Mol) Butandiol-A-diglycidylether (Araldit®DY 026) werden mit 88,11 g (1,0 Mol) Hydroxyethylvinylether und 0,17 g KOH unter Stickstoffatmosphäre auf 120 °C erhitzt. Dann wird die Mischung 12 Stunden lang bei dieser Temperatur und danach 48 Stunden bei 150 °C gerührt.
B) 150 g (0,39 Mol) des so erhaltenenen Divinylethers werden mit 569,6 g (5,96 Mol) Epichlorhydrin und 5,1 g einer 50%-igen Tetramethylammoniumchloridlösung bei 70 °C eine Stunde lang gerührt. Dann werden unter reduziertem Druck 59,2 g einer 50%-igen NaOH-Lösung zugetropft (90 mbar). Unter Abscheidung des Wassers wird gemäss Beispiel 1 aufgearbeitet. Es werden 159,5 g (83,5% der theoretischen Ausbeute) des erwünschten Produktes in Form einer farblosen Flüssigkeit erhalten, dessen Epoxidgehalt 3,52 Äquivalente/kg beträgt (86,2% der Theorie).

### Beispiel 4: Herstellung einer Vinyletherverbindung mit der Formel:

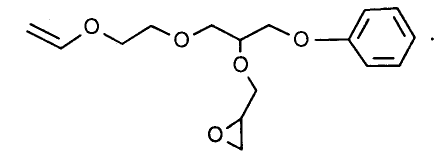

A) 158,08 g (1,0 Mol) Phenylglycidylether werden mit 88,11 g (1,0 Mol) Hydroxyethylvinylether und 0,34 g KOH gemäss Beispiel 1 umgesetzt.
B) 100 g (0,42 Mol) des so erhaltenenen Divinylethers werden mit 621,2 g (6,71 Mol) Epichlorhydrin und 5,59 g einer 50%-igen Tetramethylammoniumchloridlösung gemäss Beispiel 1 umgesetzt. Nach Zutropfen von 67,2 g einer 50%-igen NaOH-Lösung wird unter Abscheidung des Wassers gemäss Beispiel 1 aufgearbeitet. Es werden 116,48 g (94% der theoretischen Ausbeute) des erwünschten Produktes in Form einer gelblichen Flüssigkeit erhalten, dessen Epoxidgehalt 3,4 Äquivalente/kg beträgt (87% der Theorie).

### Beispiel 5: Herstellung einer Vinyletherverbindung mit der Formel:

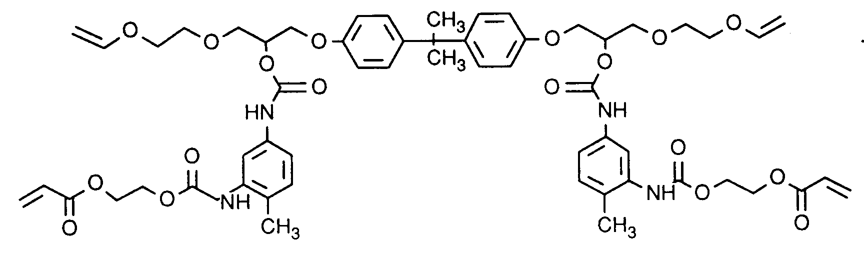

65,89 g (0,38 Mol) Toluylendiisocyanat werden mit 0,33 g 2,2'-Methylen-bis-(6-tert.-butyl-4-methylphenol) (Ralox®46) unter Rühren auf 35 °C erwärmt und 43,93 g (0,38 Mol) Hydroxyethylacrylat hinzugetropft. Die Mischung wird 8 Stunden lang bei 35 °C gerührt, bis ein Isocyanatgehalt von 3,4 Äquivalenten/kg erhalten wird. Dann werden 100g (0,19 Mol) des Divinylethers aus Beispiel 1A), gelöst in 100 Milliliter Toluol, zugetropft. Nach 4 Stunden bei 35 °C wird ein Isocyanatgehalt von 1,17 Äquivalenten/kg bestimmt. Nun werden 0,24 g Dibutylzinnlaurat hinzugefügt, und nach weiteren 2 Stunden bei 35 °C beträgt der Isocyanatgehalt 0,07 Äquivalente/kg. Das Lösungsmittel wird im Rotationverdampfer entfernt und der Rückstand unter Hochvakuum getrocknet. Es werden 200 g (95% der theoretischen Ausbeute) des erwünschten Produktes in Form eines sehr viskosen, gelben Harzes erhalten. Mit Hilfe von GPC ergibt sich eine Molekulargewicht von Mn = 1430; Mw = 6850.

### Beispiel 6: Herstellung einer Vinyletherverbindung mit der Formel:

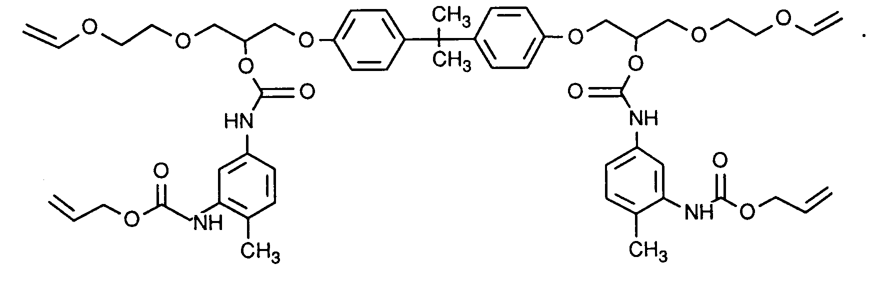

33,7 g (0,19 Mol) Toluylendiisocyanat werden mit 0,25 g Ralox®46 unter Rühren auf 35 °C erwärmt und 11,25 g (0,19 Mol) Allylalkohol hinzugetropft. Das Reaktionsgemisch wird gekühlt, damit eine (Innen)temperatur von 60 °C nicht überschritten wird. Nach 40 Minuten werden 50 Milliliter Toluol hinzugefügt und der Isocyanatgehalt der Lösung bestimmt (2,99 Äquivalente/kg). Dann wird eine Lösung von 50 g (0,097 Mol) des Divinylethers aus Beispiel 1A) in 50 Milliliter Toluol hinzugetropft und das Reaktionsgemisch etwa 24 Stunden bei 45 °C gerührt. Nach Zugabe von 0,12 g Dibutylzinnlaurat wird noch einmal 4 Stunden bei 35 °C gerührt, wobei der Isocyanatgehalt auf 0,04 Äquivalente/kg sinkt. Nach Abziehen des Lösungsmittels im Rotationsverdampfer und Trocknen des Rückstandes unter Hochvakuum erhält man 87,8 g (92% der theoretischen Ausbeute) des erwünschten Produktes in Form eines sehr viskosen, gelben Harzes (Mn = 1270; Mw = 7880; bestimmt mit GPC).

### Beispiel 7: Herstellung einer Vinyletherverbindung mit der Formel:

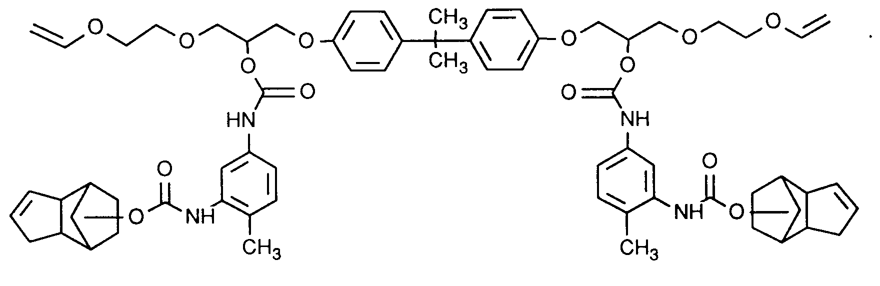

67,42 g (0,387 Mol) Toluylendiisocyanat werden mit 0,5 g Ralox®46 unter Rühren auf 35 °C erwärmt und 58,13 g (0,387 Mol) Tricyclodecanalkohol E (HOECHST) langsam hinzugetropft. Das Reaktionsgemisch wird gekühlt, damit eine (Innen)temperatur von 35 °C nicht überschritten wird. Nach 1,5 Stunden beträgt der Isocyanatgehalt der Lösung 3,08 Äquivalente/kg. Die Mischung wird mit 50 Milliliter Toluol verdünnt. Dann wird eine Lösung von 100 g (0,193 Mol) des Divinylethers aus Beispiel 1A) in 100 Milliliter Toluol hinzugetropft. Nach 8 Stunden bei 35 °C beträgt der Isocyanatgehalt 0,35 Äquivalente/kg. Nach Zugabe von 0,24 g Dibutylzinnlaurat wird noch einmal 4 Stunden bei 35 °C gerührt, wobei der Isocyanatgehalt auf 0,06 Äquivalente/kg sinkt. Nach Abziehen des Lösungsmittels im Rotationsverdampfer und Trocknen des Rückstandes unter Hochvakuum erhält man 209,5 g (92,9% der theoretischen Ausbeute) des erwünschten Produktes in Form eines viskosen, gelben Harzes (Mn = 1220; Mw = 5130; bestimmt mit GPC).

### Beispiel 8: Herstellung einer Vinyletherverbindung mit der Formel:

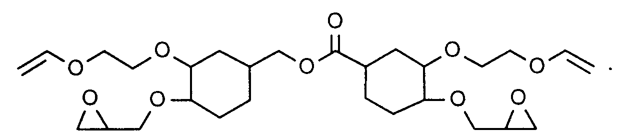

A) 137,3 g (0,5 Mol) 3,4-Epoxycyclohexylmethyl-3',4'-epoxycyclohexancarboxylat (Araldit® CY 179) werden mit 88,11 g (1,0 Mol) Hydroxyethylvinylether und 0,072 g KOH gemäss Beispiel 1 umgesetzt.
B) 150 g (0,35 Mol) des so erhaltenenen Divinylethers werden mit 518,1 g (5,60 Mol) Epichlorhydrin und 4,66 g einer 50%-igen Tetramethylammoniumchloridlösung gemäss Beispiel 1 umgesetzt. Nach Zutropfen von 56 g einer 50%-igen NaOH-Lösung wird unter Abscheidung des Wassers gemäss Beispiel 1 aufgearbeitet. Es werden 153,8 g (80,9% der theoretischen Ausbeute) des erwünschten Produktes in Form einer bräunlichen Flüssigkeit erhalten, dessen Epoxidgehalt 2,34 Äquivalente/kg beträgt (63,1% der Theorie).

### Beispiel 9: Herstellung einer Vinyletherverbindung mit der Formel:

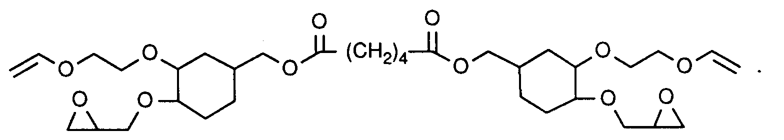

A) 161,1 g (0,4 Mol) eines cycloaliphatischen Epoxidharzes der Formel 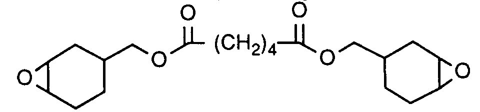 (Araldit® CY 177) werden mit 70,5 g (0,8 Mol) Hydroxyethylvinylether und 0,058 g KOH gemäss Beispiel 1 umgesetzt.
B) 150 g (0,23 Mol) des so erhaltenenen Divinylethers werden mit 340,47 g (3,68 Mol) Epichlorhydrin und 3,06 g einer 50%-igen Tetramethylammoniumchloridlösung gemäss Beispiel 1 umgesetzt. Nach Zutropfen von 36,8 g einer 50%-igen NaOH-Lösung wird unter Abscheidung des Wassers gemäss Beispiel 1 aufgearbeitet. Es werden 113,7 g (75,5% der theoretischen Ausbeute) des erwünschten Produktes in Form einer bräunlichen Flüssigkeit erhalten, dessen Epoxidgehalt 1,04 Äquivalente/kg beträgt (34,1% der Theorie).

### Beispiel 10: Herstellung einer Vinyletherverbindung der Formel:

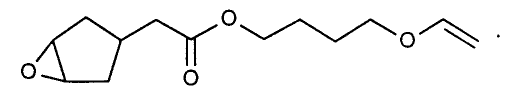

A) 43,46 g (0,31 Mol) Cyclopentenylessigsäuremethylester werden in 80 ml Chloroform gelöst. Nach Hinzufügen von 4 g Natriumacetat werden unter Rühren 82,51 g einer 40%-igen Lösung von Peressigsäure in Essigsäure hinzugetropft, wobei die Temperatur auf ca. 35 °C gehalten wird. Danach wird weitere 5 Stunden bei 35 °C gerührt. Die Reaktionsmischung wird mit 5%-iger Natriumhydrogencarbonatlösung und danach zweimal mit Wasser extrahiert. Dann wird die organische Phase abgetrennt, getrocknet und restliches Peroxid mit Natriumsulfit zerstört. Nach Destillation der organischen Phase erhält man 32,3 g 3,4-Epoxycyclopentylessigsäuremethylester (66,5 % der Theorie).
B) In einem Sulfierkolben mit Rührer, Thermometer und einem Destillationsaufsatz mit Vigreux-Kolonne erhitzt man 29,87 g (0,19 Mol) dieses Epoxids mit 44,43 g (0,38 Mol) Hydroxybutylvinylether und 0,04 g Titantetraisopropyloxid unter Stickstoff zum Rückfluss. Das entstehende Methanol wird dabei laufend abdestilliert, so dass nach ca. 11 Stunden 6,7 g Methanol abgeschieden sind. Der überschüssige Hydroxybutylvinylether wird danach im Hochvakuum bei 80 °C abdestilliert. Den Rückstand löst man in Essigester, extrahiert mit einer 5%-igen Natriumhydrogencarbonatlösung und dann mit Wasser. Nach Entfernung des Lösungsmittels mit Hilfe eines Rotationsverdampfers erhält man 42,23 g des gewünschten Epoxy-vinylethers (92,5 % der Theorie (GC 98%).

### Beispiel 11: Herstellung einer Vinyletherverbindung der Formel:

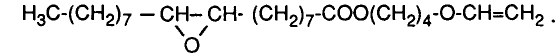

A) Gemäss einer Vorschrift von R. F. Storey, T. P. Hickey; J. Polym. Sci. A, Polym. Chem. 31 (1993), S. 1825, löst man zunächst 148,24 g (0,5 Mol) Ölsäuremethylester (technisches Isomerengemisch) in 300 ml Chloroform. Dann fügt man 255 g (0,6 Mol) 8%-iges H₂O₂, 4g (0,01 Mol) Tricaprylmethylammoniumchlorid (Aliquat®336), 8,2 g (0,025 Mol) Natriumwolframathydrat und 4,9 g (0,05 Mol) Phosphorsäure hinzu. Die Mischung wird 5 Stunden bei 60 °C gerührt. Dann wird die organische Phase abgetrennt, mit einer 5%-igen NaHCO₃-Lösung und Wasser extrahiert, getrocknet, und die restlichen Peroxide werden mit Natriumsulfit zerstört. Das Lösungsmittel wird am Rotationsverdampfer entfernt und der Rückstand 2 Stunden am Hochvakuum getrocknet. Man erhält 155,7 g eines orangebraunen Öls mit einem Epoxidgehalt von 3,03 Äquivalenten/Kilogramm (94,7% d. Th.).
B) In einem Sulfierkolben mit Rührer, Thermometer und Destillationsaufsatz werden 50 g (0,16 Mol) dieses Epoxids in 100 ml Toluol gelöst und 37,17 g (0,32 Mol) Hydroxybutylvinylether und 3,74 g Dibutylzinnoxid hinzugefügt. Die Mischung wird unter Rühren auf 105 °C erhitzt, wobei eine Mischung aus Methanol und Toluol abdestilliert. Man setzt die Umsetzung fort, bis im GC kein Edukt mehr nachweisbar ist (ca. 11 Stunden). Nach Entfernen des überschüssigen Hydroxybutylvinylethers wird der Rückstand in Dichlormethan gelöst und mit NaHCO₃ und Wasser wie oben beschrieben extrahiert. Nach Entfernung des Lösungsmittels im Rotationsverdampfer und Trocknung des Rückstands am Hochvakuum erhält man 63 g des gewünschten Produktes (99,2% d. Th; GC ca. 94%).

### Beispiel 12: Herstellung einer Vinyletherverbindung der Formel:

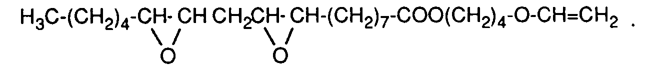

A) Analog zu Beispiel 11 A) werden 143,9 g (0,49 Mol) Linolsäuremethylester mit 1008,72 g (2,39 Mol) 8%-igem H₂O₂ unter Zusatz von 8,86 g Aliquat®336, 16,06 g Natriumwolframat und 9,59 g Phosphorsäure umgesetzt. Nach Entfernen des Lösungsmittels erhält man ein gelbes Öl in einer Ausbeute von 154,2 g (96,4 % d. Th.) mit einem Epoxidgehalt von 4,15 Äquivalenten/Kilogramm (67,7 % d. Th.).
B) Analog zu Beispiel 10 B) werden 148,76 g (0,456 Mol) dieses Epoxids mit 76,99 g (0,68 Mol) Hydroxybutylvinylether und 0,21 g Titaniumtetraisopropyloxid umgesetzt. Nach ca. 11 Stunden ist die Methanolabscheidung beendet. Man isoliert 179,27 g (95,5 % d. Th.) des gewünschten Produktes.

### Beispiel 13: Herstellung einer Vinyletherverbindung der Formel:

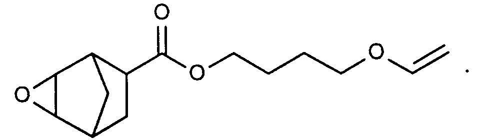

A) Analog zu Beispiel 11 A) werden 120 g (0,788 Mol) 5-Carbomethoxy-2-norbornen mit 807,8 g (1,9 Mol) 8%-igem H₂O₂ unter Zusatz von 6,37 g Aliquat®336, 13,7 g Natriumwolframat und 7,12 g Phosphorsäure umgesetzt. Nach Destillation unter reduziertem Druck erhält man 56,8 g (42,9 % d. Th.) Epoxid.
B) Analog zu Beispiel 10 B) werden 56 g (0,33 Mol) dieses Epoxids mit 77,4 g (0,66 Mol) Hydroxybutylvinylether und 0,2 g Titaniumtetraisopropyloxid umgesetzt. Nach Entfernung des überschüssigen Hydroxybutylvinylethers und Extraktion der organischen Phase wie in Beispiel 10 B) beschrieben isoliert man 92 g einer gelblichen Flüssigkeit. Eine Destillation von 20 g dieser Flüssigkeit ergibt 14,9 g des gewünschten Epoxy-Vinylethers (Siedepunkt 116 - 118 °C bei 0,5 mbar Druck).

### Beispiel 14: Herstellung einer Vinyletherverbindung der Formel:

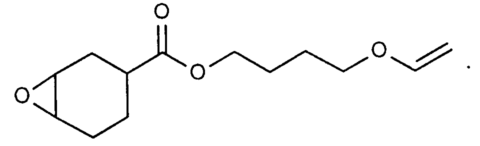

A) Analog zu Beispiel 10 A) werden 50 g (0,36 Mol) Cyclohex-3-en-carbonsäuremethylester mit 171,1 g Peressigsäure in Essigsäure umgesetzt. Nach Destillation erhält man 38,9 g (69 % d. Th.) Epoxid.
B) Analog zu Beispiel 10 B) werden 38 g (0,243 Mol) dieses Epoxids mit 56,6 g (0,486 Mol) Hydroxybutylvinylether und 0,13 g Titaniumtetraisopropyloxid umgesetzt. Man isoliert 92 g (80% d. Th.) des gewünschten Produkts in Form einer orangen Flüssigkeit.

### Beispiel 15: Herstellung einer Vinyletherverbindung der Formel:

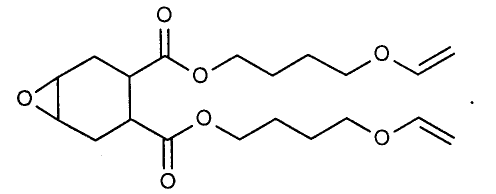

A) Analog zu Beispiel 10 A) werden 168,80 g (0,85 Mol) Tetrahydrophthalsäuredimethylester mit 230,1 g einer 40%-igen Lösung von Peressigsäure in Essigsäure und 10 g Natriumacetat umgesetzt. Man erhält 114,2 g (62,7 % d. Th.) Epoxid mit einem Epoxidgehalt von 3,39 Äquivalente/Kilogramm (72,7% d. Th.).
B) Analog zu Beispiel 11 B) werden 108,38 g (0,506 Mol) dieses Epoxids mit 232,32 g (2 Mol) Hydroxybutylvinylether unter Zusatz von 6,81 g Dibutylzinnoxid so lange umgesetzt, bis im GC kein Edukt mehr nachweisbar ist (ca. 11 Stunden). Nach Entfernen des überschüssigen Hydroxybutylvinylethers wird der Rückstand mit Wasser extrahiert und man erhält 150,12 g des gewünschten Produktes (98,2% d. Th.).

### Beispiel 16:

Die folgenden Komponenten werden unter Rühren bei 60 °C vermischt, bis eine klare Lösung entsteht:
- 48,4 g: 3,4-Epoxycyclohexylmethyl-3',4'-epoxycyclohexancarboxylat (Araldit®CY 179);
- 18 g: Butandioldiglycidylether (Araldit®DY 026);
- 20 g: der Vinyletherverbindung aus Beispiel 3;
- 6 g: Dipentaerythritolpentaacrylat (Sartomer®399);
- 6 g: Bisphenol-A-diglycidyldiacrylat (Novacure®3700);
- 0,8 g: 1-Hydroxycyclohexylphenylketon (Irgacure®184);
- 0,8 g: eines Cyracure® UVI 6974

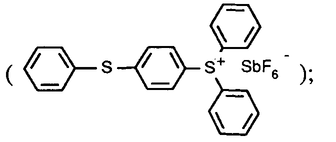

Die Viskosität der Mischung beträgt 121 mPa·s (30 °C).
Mit Hilfe eines He/Cd-Lasers werden durch Bestrahlung (Bestrahlungsenergie von 80 mJ/cm²) Formkörper der Abmessung 45,7 x 0,38 x 0,5 Millimeter hergestellt. Direkt nach der Bestrahlung weisen diese Formkörper (die sogenannten Grünlinge) folgende Eigenschaften auf:
Elastizitätsmodul (E-Modul) gemäss ISO R 527 = 544 MPa;
Reissdehnung gemäss ISO R 527, bestimmt mit der Zutestmaschine Lloyd®500 der Firma Lloyd = 23,3%.

Zur vollständigen Aushärtung werden die Grünlinge 30 Minuten mit UV-Licht bestrahlt und danach 30 Minuten auf eine Temperatur von 130 °C erwärmt. Danach werden folgende Eigenschaften gemessen:
Elastizitätsmodul = 2586 MPa;
Reissdehnung = 2%;
Schlagzähigkeit gemäss ISO 179/1D = 8,7 kJ/cm²;
Curl-Faktor (Formkörper der Bauart "Weave" gemäss P. Jacobs, Rapid Prototyping + Manufacturing, Fundamentals of Stereolitography, Soc. of Manufact. Engineers, 1992, p. 256) = -0,12.

### Beispiel 17:

Die folgenden Komponenten werden unter Rühren bei 60 °C vermischt, bis eine klare Lösung entsteht:
- 48,4 g: 3,4-Epoxycyclohexylmethyl-3',4'-epoxycyclohexancarboxylat (Araldit®CY 179);
- 18 g: der Vinyletherverbindung aus Beispiel 3;
- 20 g: eines trifunktionellen, hydroxylterminierten Polycaprolactons (Tone®0301);
- 6 g: Dipentaerythritolpentaacrylat (Sartomer®399);
- 6 g: Bisphenol-A-diglycidyldiacrylat (Novacure®3700);
- 0,8 g: 1-Hydroxycyclohexylphenylketon (Irgacure®184);
- 0,8 g: eines Triarylsulfoniumhexafluoroantimonatinitiators (Cyracure® UVI 6974);

Die Viskosität der Mischung beträgt 387 mPa·s (30 °C).
Mit Hilfe eines He/Cd-Lasers werden durch Bestrahlung (Bestrahlungsenergie von 80 mJ/cm²) Formkörper der Abmessung 45,7 x 0,38 x 0,51 Millimeter hergestellt. Direkt nach der Bestrahlung weisen diese Formkörper (die sogenannten Grünlinge) folgende Eigenschaften auf:
Elastizitätsmodul (E-Modul) = 90,8 MPa;
Reissdehnung = 70%.

Zur vollständigen Aushärtung werden die Grünlinge 30 Minuten mit UV-Licht bestrahlt und 30 Minuten auf eine Temperatur von 130 °C erwärmt. Danach werden folgende Eigenschaften gemessen:
Elastizitätsmodul = 2663 MPa;
Reissdehnung = 16,8%;
Schlagzähigkeit gemäss ISO 179/1D = 42 kJ/cm²;
Curl-Faktor (Formkörper der Bauart "Weave") = 0,013.

### Beispiel 18:

Die folgenden Komponenten werden unter Rühren bei 60 °C vermischt, bis eine klare Lösung entsteht:
- 48,4 g: 3,4-Epoxycyclohexylmethyl-3',4'-epoxycyclohexancarboxylat (Araldit®CY 179);
- 18 g: der Vinyletherverbindung aus Beispiel 4;
- 20 g: eines trifunktionellen, hydroxylterminierten Polycaprolactons (Tone®0301);
- 6 g: Dipentaerythritolpentaacrylat (Sartomer®399);
- 6 g: Bisphenol-A-diglycidyldiacrylat (Novacure®3700);
- 0,8 g: 1-Hydroxycyclohexylphenylketon (Irgacure®184);
- 0,8 g: eines Triarylsulfoniumhexafluoroantimonatinitiators (Cyracure® UVI 6974);

Die Viskosität der Mischung beträgt 281 mPa·s (30 °C).
Mit Hilfe eines He/Cd-Lasers werden durch Bestrahlung (Bestrahlungsenergie von 160 mJ/cm²) Formkörper der Abmessung 45,7 x 0,38 x 0,51 Millimeter hergestellt. Direkt nach der Bestrahlung weisen diese Formkörper (die sogenannten Grünlinge) folgende Eigenschaften auf:
Elastizitätsmodul (E-Modul) = 244 MPa;
Reissdehnung = 86%.

Zur vollständigen Aushärtung werden die Grünlinge 30 Minuten mit UV-Licht bestrahlt und 30 Minuten auf eine Temperatur von 130 °C erwärmt. Danach werden folgende Eigenschaften gemessen:
Elastizitätsmodul = 3083 MPa;
Reissdehnung = 8,1%;
Schlagzähigkeit gemäss ISO 179/1D = 31,9 kJ/cm²;
Curl-Faktor (Formkörper der Bauart "Weave") = 0,0017.

### Beispiel 19:

Die folgenden Komponenten werden unter Rühren bei 60 °C vermischt, bis eine klare Lösung entsteht:
- 48,4 g: 3,4-Epoxycyclohexylmethyl-3',4'-epoxycyclohexancarboxylat (Araldit®CY 179);
- 18 g: der Vinyletherverbindung aus Beispiel 10;
- 20 g: eines trifunktionellen, hydroxylterminierten Polycaprolactons (Tone®0301);
- 6 g: Dipentaerythritolpentaacrylat (Sartomer®399);
- 6 g: Bisphenol-A-diglycidyldiacrylat (Novacure®3700);
- 0,8 g: 1-Hydroxycyclohexylphenylketon (Irgacure®184);
- 0,8 g: eines Triarylsulfoniumhexafluoroantimonatinitiators (Cyracure® UVI 6974);

Die Viskosität der Mischung beträgt 224 mPa·s (30 °C).

Mit Hilfe eines He/Cd-Lasers werden durch Bestrahlung Formkörper der Abmessung 40 x 4,25 x 2,5 Millimeter hergestellt (Baustil Weave). Direkt nach der Bestrahlung weisen diese Formkörper einen (E-Modul aus dem Biegeversuch (gemäss ISO 178/75) von 436 MPa auf.

Zur vollständigen Aushärtung werden die Grünlinge 60 Minuten mit UV-Licht bestrahlt und 30 Minuten auf eine Temperatur von 100 °C erwärmt. Danach werden folgende Eigenschaften gemessen:
Elastizitätsmodul (gemäss ISO R 527) = 2718 MPa;
Zugfestigkeit (gemäss ISO R 527) = 63 MPa;
Reissdehnung = 7,3%;
Schlagzähigkeit (gemäss DIN 52453) = 22,5 kJ/cm².

### Beispiel 20:

- 48,4 g: 3,4-Epoxycyclohexylmethyl-3',4'-epoxycyclohexancarboxylat (Araldit®CY 179);
- 7 g: der Vinyletherverbindung aus Beispiel 15;
- 20 g: eines glycidilisierten Ricinusöls (Heloxy®505);
- 18 g: eines Polyester-polyols (Desmophen®850);
- 5 g: Dipentaerythritolpentaacrylat (Sartomer®399);
- 0,8 g: Irgacure®184;
- 0,8 g: eines Triarylsulfoniumhexafluoroantimonatinitiators (Cyracure® UVI 6974);

Die Viskosität der Mischung beträgt 600 mPa·s (30 °C).

Nach der Laserhärtung weist das Material einen Elastizitätsmodul (E-Modul) von 201 MPa auf.

Zur vollständigen Aushärtung wird das Material ebenfalls 60 Minuten mit UV-Licht bestrahlt und 30 Minuten auf eine Temperatur von 100 °C erwärmt. Danach werden folgende Eigenschaften gemessen:
Elastizitätsmodul = 2043 MPa;
Zugfestigkeit = 47,7 MPa;
Reissdehnung = 16%;
Schlagzähigkeit = 37 kJ/m²;
Der Curl-Faktor eines mit dem Baustil Weave gebauten Probekörpers betrug 0%.

### Beispiel 21:

Die folgenden Komponenten werden unter Rühren bei 60 °C vermischt:
- 48,4 g: 3,4-Epoxycyclohexylmethyl-3',4'-epoxycyclohexancarboxylat (Araldit®CY 179);
- 12 g: der Vinyletherverbindung aus Beispiel 15;
- 20 g: eines glycidilisierten Ricinusöls (Heloxy®505);
- 13 g: eines Polyester-polyols (Desmophen®850);
- 5 g: eines trifunktionellen, hydroxylterminierten Polycaprolactons (Tone®0301);
- 1,6 g: eines Triarylsulfoniumhexafluoroantimonatinitiators (Cyracure® UVI 6974);

Die Viskosität der Mischung beträgt 400 mPa·s (30 °C).

Nach der Laserhärtung weist das Material einen Elastizitätsmodul (E-Modul) von 348,4 MPa auf.

Zur vollständigen Aushärtung wird das Material ebenfalls 60 Minuten mit UV-Licht bestrahlt und 30 Minuten auf eine Temperatur von 100 °C erwärmt. Danach werden folgende Eigenschaften gemessen:
Elastizitätsmodul = 719 MPa;
Zugfestigkeit = 28,8 MPa;
Reissdehnung = 55,7%;
Der Curl-Faktor eines mit dem Baustil Weave gebauten Probekörpers betrug -4%.

### Beispiel 22:

Die folgenden Komponenten werden unter Rühren bei 60 °C vermischt:
- 48 g: 3,4-Epoxycyclohexylmethyl-3',4'-epoxycyclohexancarboxylat (Araldit®CY 179);
- 18 g: der Vinyletherverbindung aus Beispiel 3;
- 6 g: Bisphenol-A-diglycidyldiacrylat (Novacure®3700);
- 6 g: Dipentaerythritolpentaacrylat (Sartomer®399);
- 20 g: eines trifunktionellen, hydroxylterminierten Polycaprolactons (Tone®0301);
- 1,6 g: Diphenyliodonium-hexafluoroarsenat 0,8 g Irgacure® 184;

Die Viskosität der Mischung beträgt 450 mPa·s (39 °C).

Mit Hilfe eines He/Cd-Lasers werden durch Bestrahlung Formkörper der Abmessung 40 x 4,3 x 3,8 Millimeter hergestellt. Direkt nach der Bestrahlung weisen diese Formkörper einen E-Modul aus dem Biegeversuch (ISO 178/75) von 144,9 MPa auf.

Zur vollständigen Aushärtung werden die Grünlinge 60 Minuten mit UV-Licht bestrahlt und 30 Minuten auf 100 °C erwärmt. Danach werden folgende Eigenschaften gemessen:
E-Modul = 2811 MPa;
Zugfestigkeit = 62,1 MPa;
Reissdehnung = 7,5%;

## Patentansprüche

1. Chemische Verbindung mit mindestens einer Vinylethergruppe, die ausserdem mindestens eine weitere funktionelle Gruppe ausgewählt aus Acrylat-, Methacrylat-, Epoxid-, Alkenyl-, Cycloalkenyl- sowie Vinylarylgruppen im Molekül aufweist,
dadurch gekennzeichnet,
dass es sich um eine Verbindung der folgenden Formel handelt:
[H₂C = CH―O⁆_{z}A
wobei die in dieser und den weiteren Formeln verwendeten Symbole die nachfolgende Bedeutung haben:
A ein z-wertiger Rest ausgewählt aus den Resten der nachfolgenden Formeln (1), (2), (3) und (4) 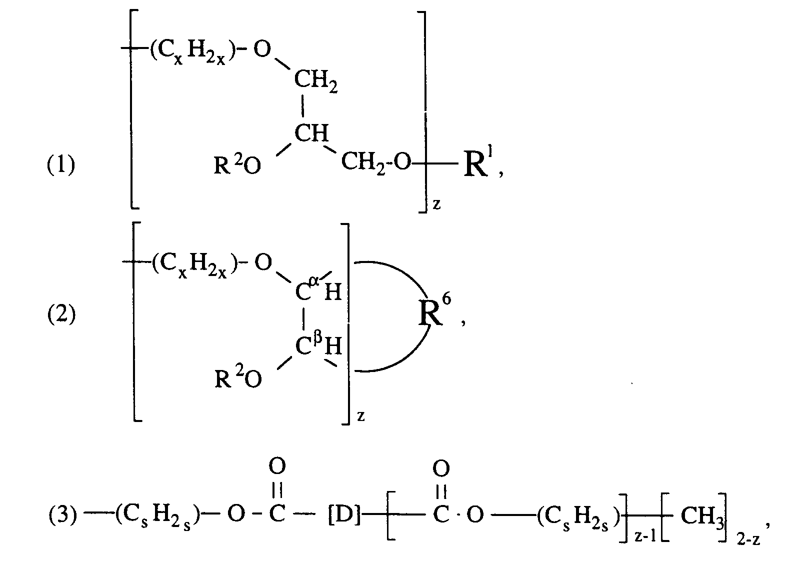 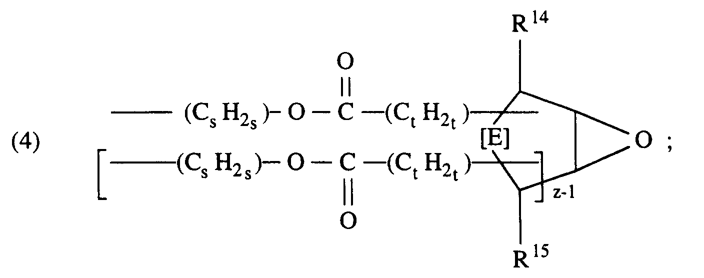
[D] eine Gruppe der Formel 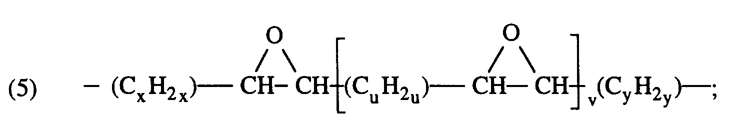
[E] eine C₁- oder eine C₂-Alkylengruppe;
R⁰ ein Wasserstoffatom oder eine Methylgruppe;
R¹ ein z-wertiger Rest ausgewählt aus
aliphatischen, cycloaliphatischen, aromatischen,
aliphatisch-aromatischen und aliphatisch-cycloaliphatischen Resten sowie Polyoxyalkylenresten;
R² ein Rest ausgewählt aus den Resten der Formeln 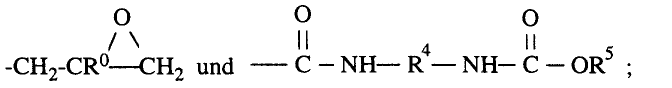
R⁴ eine Gruppe ausgewählt aus den Gruppen der Formeln 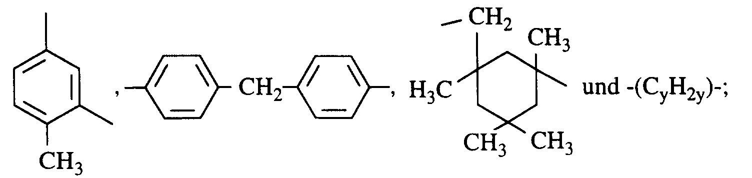
R⁵ eine Gruppe ausgewählt aus den Gruppen der Formeln 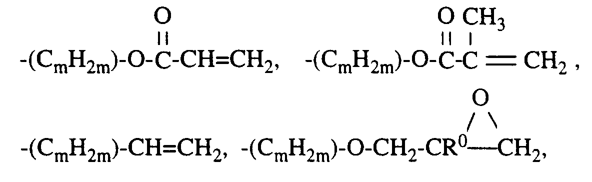 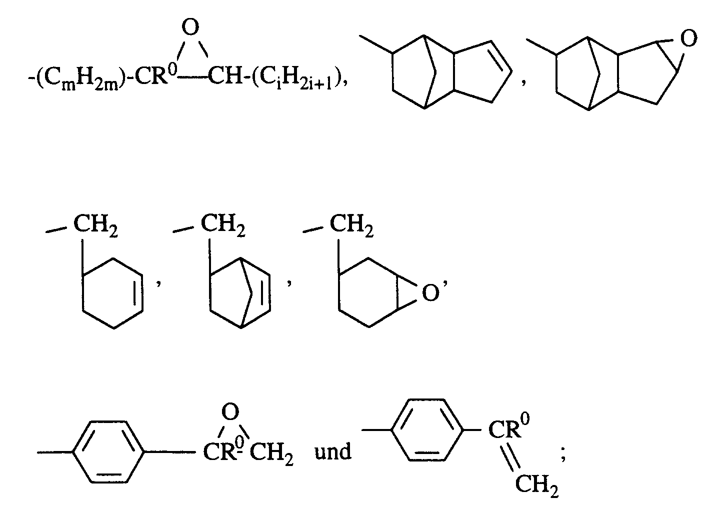
R⁶ eine (2·z)-wertige organische Gruppe, die jeweils mit den Kohlenstoffatomen C^{α} und C^{β} jeder der z Gruppen der Formel 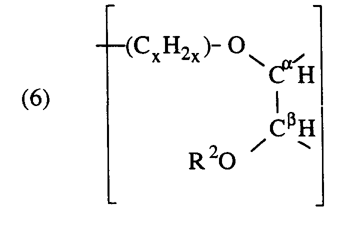 in einem Rest der Formel (2) einen cycloaliphatischen Ring mit mindestens 5 Kohlenstoffatomen bildet;
R¹⁴ und R¹⁵ jeweils ein Wasserstoffatom oder, falls [E] eine C₂-Alkylengruppe bedeutet, jeweils ein Wasserstoffatom oder zusammen eine Methylengruppe;
i eine ganze Zahl von 0 bis 20;
m eine ganze Zahl von 1 bis 20;
s eine ganze Zahl von 2 bis 10;
t eine ganze Zahl von 0 bis 10;
u in den einzelnen Einheiten der Formel 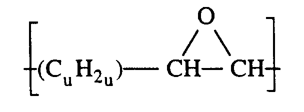 in Formel (5) unabhängig voneinander jeweils eine ganze Zahl von 1 bis 20;
v eine ganze Zahl von 0 bis 4;
x sowie y unabhängig voneinander jeweils eine ganze Zahl von 2 bis 20 und
z die Zahl 1 oder 2.

2. Verbindung gemäss Anspruch 1 mit der Formel 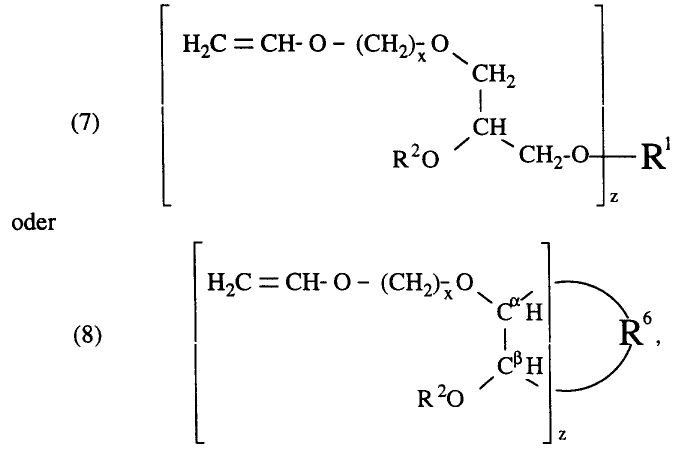 worin R¹, R², R⁶, x, z die gleiche Bedeutung wie in Anspruch 1 haben.

3. Verbindungen nach Anspruch 1 oder 2, worin
R¹ einen z-wertigen Rest darstellt, ausgewählt aus
a) aliphatischen Resten mit 2 bis 20 Kohlenstoffatomen,
b) cycloaliphatischen sowie aromatischen Resten mit
jeweils 6 bis 14 Kohlenstoffatomen,
c) aliphatisch-aromatischen sowie aliphatisch-cycloaliphatischen Resten mit jeweils 7 bis 25 Kohlenstoffatomen und
d) Polyoxyalkylenresten der Formeln
R⁷-[OC_{g}H_{2g}]ₙ- und -(C_{g}H_{2g})-[OC_{g}H_{2g}]ₙ₋₁-,
worin
R⁷ eine Alkylgruppe mit 1 bis 8 Kohlenstoffatomen,
g eine Zahl von 1 bis 8 entsprechend der durchschnittlichen Anzahl von Kohlenstoffatomen einer Alkyleneinheit des Polyoxyalkylenrestes und
n eine ganze Zahl von 2 bis 20 bedeutet,
und der Rest R¹ unsubstituiert ist oder zusätzlich einen oder mehrere Substituenten aufweist, die
im Falle eines aliphatischen Restes R¹ ausgewählt sind aus
C₁-C₄-Alkoxy- und Halogen-Substituenten;
und
im Falle der andersartigen Reste R¹ausgewählt aus
C₁-C₄-Alkyl-, C₁-C₄-Alkoxy- und Halogen-Substituenten.

4. Verbindungen nach Anspruch 3, worin
R¹ einen Rest ausgewählt aus den Resten der Formeln 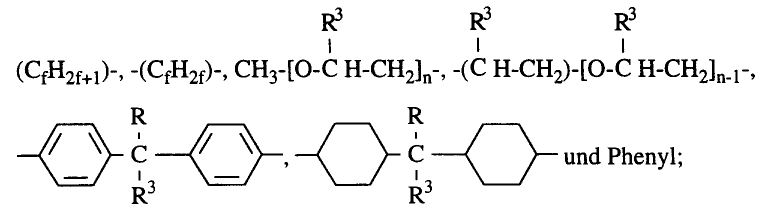 sowie
R und R³ unabhängig voneinander jeweils ein Wasserstoffatom oder Methyl und
f eine ganze Zahl von 2 bis 20
bedeuten.

5. Verbindungen nach Anspruch 4, worin
R¹ einen Rest ausgewählt aus den Resten der Formeln 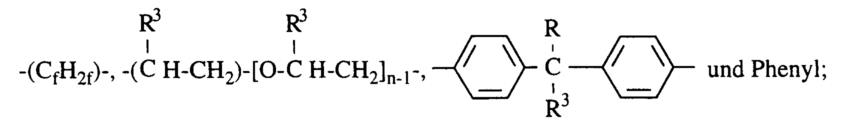
R⁴ eine Gruppe ausgewählt aus den Gruppen der Formeln 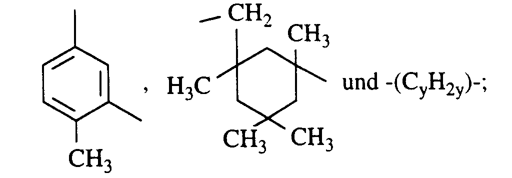
R⁵ eine Gruppe ausgewählt aus den Gruppen der Formeln 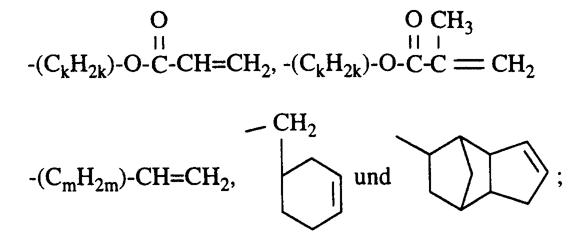 sowie
R und R³ beide entweder ein Wasserstoffatom oder Methyl;
k eine ganze Zahl von 2 bis 10 bedeuten
und für den Index
m eine Obergrenze von 10 gilt.

6. Verbindungen nach Anspruch 5, worin
R¹ einen Rest ausgewählt aus
einem Alkylenrest mit 2 bis 4 Kohlenstoffatomen, einem Phenylrest und einem Rest der Formel 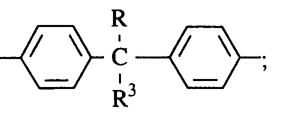
R⁴ eine Gruppe der Formel 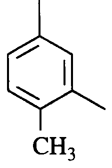 und
R⁵ eine Gruppe ausgewählt aus den Gruppen der Formeln 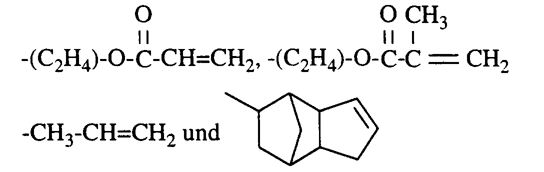
bedeuten.

7. Verbindungen nach Anspruch 1 oder 2, worin
R⁶ eine organische Gruppe mit 3 bis 50 Kohlenstoffatomen ist.

8. Verbindungen nach Anspruch 7, worin
z 1 ist und
R⁶ zusammen mit den Kohlenstoffatomen C^{α} und C^{β} der Gruppe der Formel (6) einen Cycloalkylrest mit 5 bis 7 Ringkohlenstoffatomen bildet, an den gegebenenfalls ein weiterer Cycloalkylrest mit 5 bis 7 Ringkohlenstoffatomen annelliert ist.

9. Verbindungen nach Anspruch 8, worin
R⁶ zusammen mit den Kohlenstoffatomen C^{α} und C^{β} der Gruppe der Formel (6) einen Cyclopentyl- oder Cyclohexylrest bildet.

10. Verbindungen nach Anspruch 7, worin
R⁶ eine Gruppe der Formel
R⁸-[G]-R⁹
darstellt, worin
R⁸ zusammen mit den Kohlenstoffatomen C^{α} und C^{β} einer Gruppe der Formel (6) einen Cycloalkylrest mit 5 bis 7 Ringkohlenstoffatomen bildet,
an den gegebenenfalls ein weiterer Cycloalkylrest
mit 5 bis 7 Ringkohlenstoffatomen annelliert ist;
R⁹ entweder mit den Kohlenstoffatomen C^{α} und C^{β}einer weiteren Gruppe der Formel (6) ebenfalls einen Cycloalkylrest mit 5 bis 7 Ringkohlenstoffatomen bildet, an den gegebenenfalls ein weiterer Cycloalkylrest
mit 5 bis 7 Ringkohlenstoffatomen annelliert ist,
oder einen Cycloalkylrest mit 5 bis 7 Ringkohlenstoffatomen bedeutet,
an den gegebenenfalls ein weiterer Cycloalkylrest
mit 5 bis 7 Ringkohlenstoffatomen annelliert ist; und
[G] ein Strukturelement bedeutet ausgewählt aus
einer Einfachbindung und den Gruppen der Formel 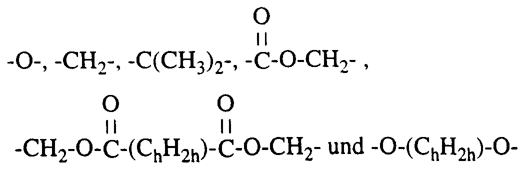 sowie
h eine ganze Zahl von 1 bis 6, insbesondere von 2 bis 4, darstellt.

11. Verbindung nach Anspruch 10 mit der Formel 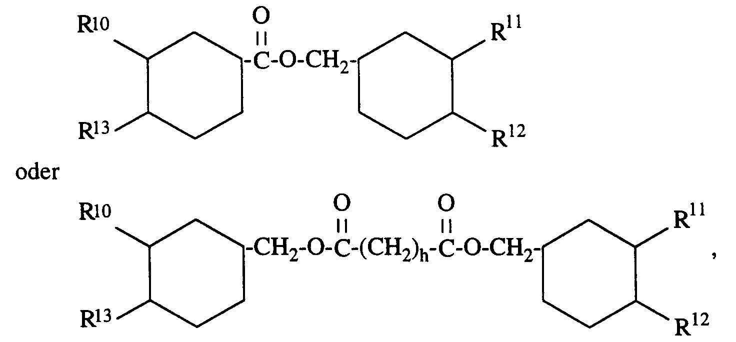 worin einer der Reste R¹⁰ oder R¹³ eine Gruppe der Formel H₂C=CH-O-(CH₂)ₓ-O- und der andere eine Gruppe der Formel R²O- und ebenso einer der Reste R¹¹ oder R¹²eine Gruppe der Formel H₂C=CH-O-(CH₂)ₓ-O- und der andere eine Gruppe der Formel R²O- und x und h unabhängig voneinander jeweils eine ganze Zähl von 2 bis 4 darstellen.

12. Verbindungen nach Anspruch 1 bis 11, worin
R² 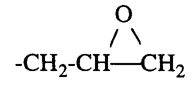 darstellt.

13. Verbindungen nach Anspruch 1, worin
A einen Rest der Formel (3) oder (4) darstellt und
s eine ganze Zahl von 2 bis 4,
t eine ganze Zahl von 0 bis 2,
u die Zahl 1,
v die Zähl 0 oder 1 und
x sowie
y eine ganze Zahl von 2 bis 10 bedeuten.

14. Verbindungen nach Anspruch 1 oder 13, worin
A einen Rest der Formel (4),
R¹⁴ und
R¹⁵ jeweils ein Wasserstoffatom sowie
z die Zahl 1 darstellen.

15. Verbindung nach Anspruch 1 oder 13 mit der Formel

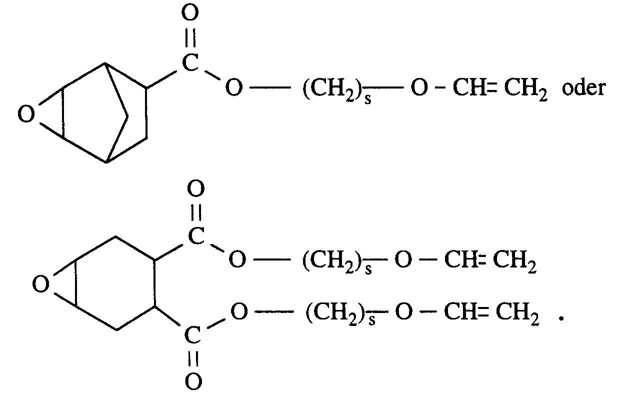

16. Verwendung von Vinyletherverbindungen nach Anspruch 1 bis 15 als eine polymerisierbare Komponente von strahlungsinduziert hartbaren Zusammensetzungen.

17. Zusammensetzung, die eine oder mehrere Vinyletherverbindungen gemäss Anspruch 1 bis 16, mindestens eine hiervon verschiedene polymerisierbare Verbindung und mindestens einen Photoinitiator für die Vernetzung der Verbindungen enthält.

18. Zusammensetzung nach Anspruch 17 enthaltend
5 bis 60 Gew.-% einer oder mehrerer Vinyletherverbindungen nach Anspruch 1 bis 15.
0 bis 40 Gew.-% mono-, di- oder mehrfunktionellen Acrylate oder Methacrylate;
30 bis 80 Gew.-% di- oder polyfunktionelle Epoxidverbindungen;
0 bis 5 Gew.-% radikalische Photoinitiatoren:
0,5 bis 5 Gew.-% kationische Photoinitiatoren;
0 bis 40 Gew.-% hydroxylterminierte Polyether oder Polyester; und
0 bis 10 Gew.-% eines oder mehrerer Additive.

19. Verfahren zur Herstellung eines gehärteten Produkts, bei dem man eine Zusammensetzung nach einem der Ansprüche 17 oder 18 mit aktinischer Strahlung behandelt.

20. Verfahren zur Herstellung von dreidimensionalen Gegenständen aus einer Zusammensetzung nach einem der Ansprüche 17 oder 18 mittels Stereolithographie, umfassend einen Schritt, bei dem die Oberfläche einer Schicht aus der Zusammensetzung ganzflächig oder in einem vorbestimmten Muster mit einer UV/VIS-Lichtquelle bestrahlt wird, so dass sich in den bestrahlten Bereichen eine Schicht in einer gewünschten Schichtdicke verfestigt, dann eine neue Schicht aus der Zusammensetzung auf der verfestigten Schicht gebildet wird, die ebenfalls ganzflächig oder in einem vorbestimmten Muster bestrahlt wird, und wobei durch wiederholtes Beschichten und Bestrahlen dreidimensionale Gegenstände aus mehreren aneinander haftenden, verfestigten Schichten erhalten werden.

## Claims

1. A chemical compound having at least one vinyl ether group which also contains in the molecule at least one further functional group selected from acrylate, methacrylate, epoxy, alkenyl, cycloalkenyl and vinylaryl groups,
wherein it is a compound of the following formula
[H₂C = CH―O⁆_{z}A
the symbols used in that formula and in the formulae below having the following definitions:
A is a z-valent radical selected from the radicals of the following formulae (1), (2), (3) and (4) 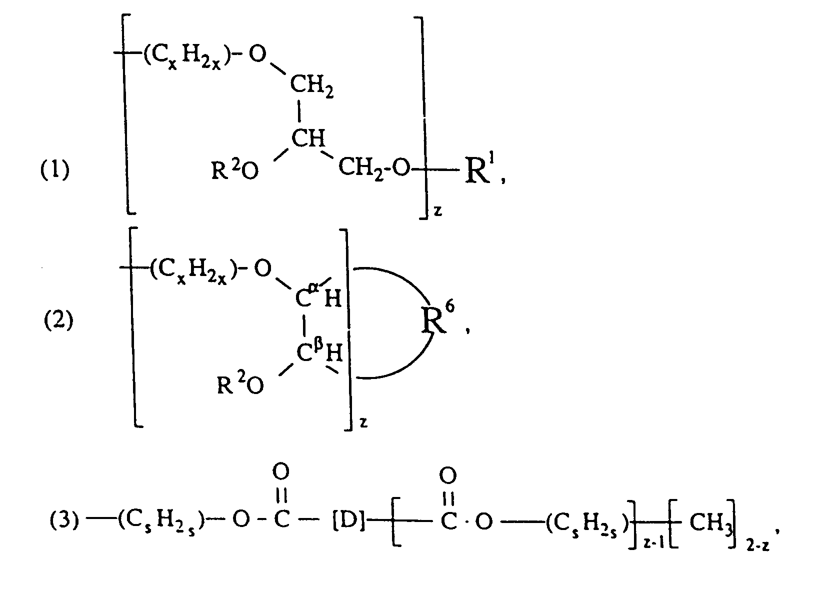 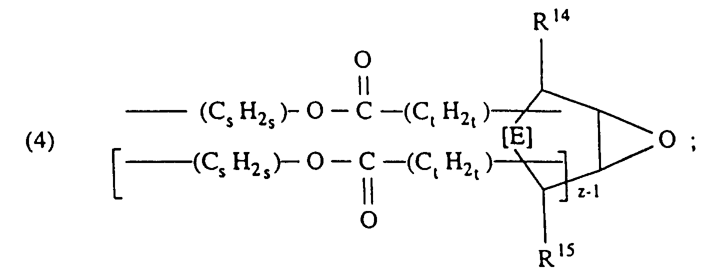
[D] is a group of the formula 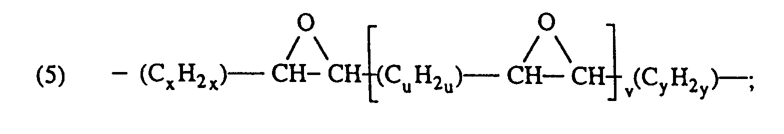
[E] is a C₁ or a C₂-alkylene group;
R⁰ is a hydrogen atom or a methyl group;
R¹ is a z-valent radical selected from aliphatic, cycloaliphatic, aromatic, aliphatic-aromatic and aliphatic-cycloaliphatic radicals and polyoxyalkylene radicals;
R² is a radical selected from the radicals of the formulae 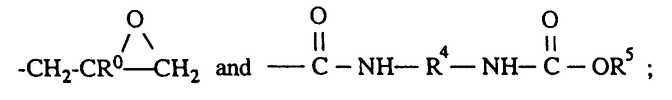
R⁴ is a group selected from the groups of the formulae 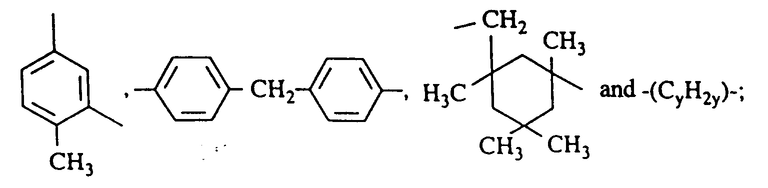
R⁵ is a group selected from the groups of the formulae 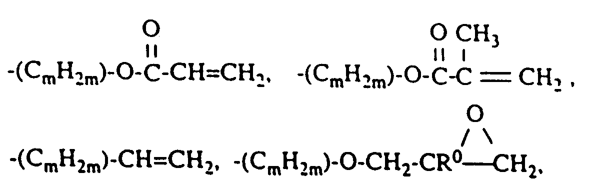 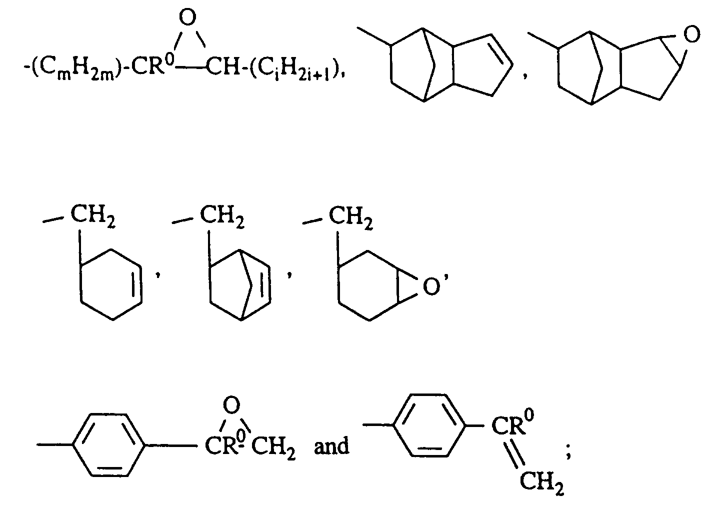
R⁶ is a (2·z)-valent organic group which, together with the carbon atoms C^{α} and C^{β} of each of the z groups of the formula 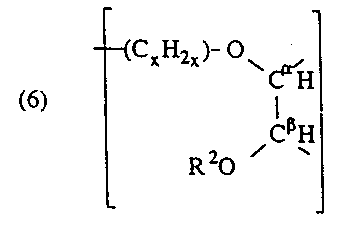 in a radical of formula (2), forms a cycloaliphatic ring having at least 5 carbon atoms;
R¹⁴ and
R¹⁵ are each a hydrogen atom or, when [E] is a C₂alkylene group, are each a hydrogen atom or together form a methylene group;
i is an integer from 0 to 0;
m is a integer from 1 to 20;
s is a integer from 2 to 10;
t is a integer from 0 to 10;
u in the individual units of the formula 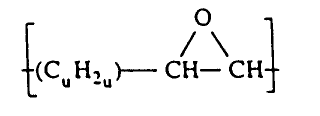 in formula (5) are independently of one another an integer from 1 to 20;
v is an integer from 0 to 4;
x and
y are independently of one another an integer from 2 to 20, and
z is the number 1 or 2.

2. A compound according to claim 1 having the formula 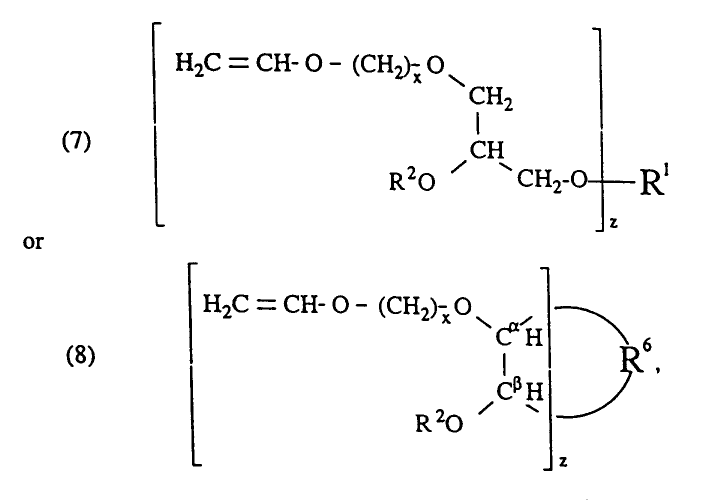 wherein R¹, R², R⁶, x and z are as defined in claim 1.

3. A compound according to claim 1 or 2 wherein
R¹ is a z-valent radical selected from
a) aliphatic radicals having from 2 to 20 carbon atoms,
b) cycloaliphatic and aromatic radicals each having from 6 to 14 carbon atoms,
c) aliphatic-aromatic and aliphatic-cycloaliphatic radicals each having from 7 to 25 carbon atoms, and
d) polyoxyalkylene radicals of the formulae R⁷-[OC_{g}H_{2g}]ₙ and -(C_{g}H_{2g})-[OC_{g}H_{2g}]ₙ₋₁ wherein
R⁷ is an alkyl group having from 1 to 8 carbon atoms,
g is a number from 1 to 8 corresponding to the average number of carbon atoms of an alkylene unit of the polyoxyalkylene radical and
n is an integer from 2 to 20,
and the radical R¹ is unsubstituted or in addition may have one or more substituents which
in the case of an aliphatic radical R¹ are selected from
C₁-C₄alkoxy and halogen substituents; and
in the case of other types of radical R¹ are selected from
C₁-C₄alkyl, C₁-C₄alkoxy and halogen substituents.

4. A compound according to claim 3 wherein
R¹ is a radical selected from the radicals of the formulae 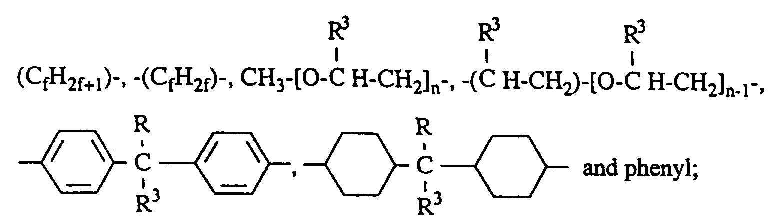 and
R and R³ are independently of one another a hydrogen atom or methyl and
f is an integer from 2 to 20.

5. A compound according to claim 4 wherein
R¹ is a radical selected from the radicals of the formulae 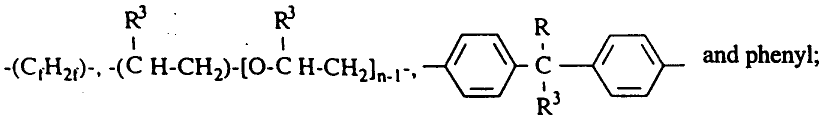
R⁴ is a group selected from the groups of the formulae 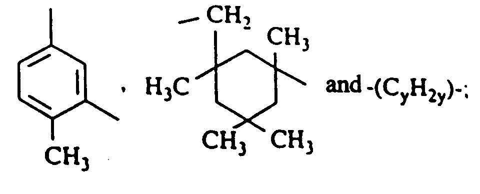
R⁵ is a group selected from the groups of the formulae 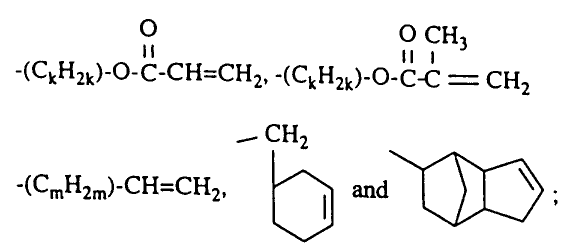 and
R and R³ are both either a hydrogen atom or methyl; and
k is an integer from 2 to 10, and the index
m has an upper limit of 10.

6. A compound according to claim 5 wherein
R¹ is a radical selected from
an alkylene radical having from 2 to 4 carbon atoms, a phenyl radical and a radical of the formula 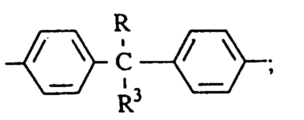
R⁴ is a group of the formula 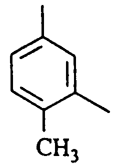 and
R⁵ is a group selected from the groups of the formulae

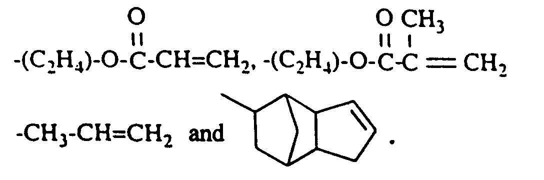

7. A compound according to claim 1 or 2 wherein
R⁶ is a organic group having from 3 to 50 carbon atoms.

8. A compound according to claim 7 wherein
z is 1 and
R⁶ together with the carbon atoms C^{α} and C^{β} of the group of formula (6) forms a cycloalkyl radical having from 5 to 7 ring carbon atoms to which a further cycloalkyl radical having from 5 to 7 ring carbon atoms may have been fused.

9. A compound according to claim 8 wherein
R⁶ together with the carbon atoms C^{α} and C^{β} of the group of formula (6) forms a cyclopentyl or cyclohexyl radical.

10. A compound according to claim 7 wherein
R⁶ is a group of the formula R⁸-[G]-R⁹ wherein
R⁸ together with the carbon atoms C^{α} and C^{β} of a group of formula (6) forms a cycloalkyl radical having from 5 to 7 ring carbon atoms to which a further cycloalkyl radical having from 5 to 7 ring carbon atoms may have been fused;
R⁹ either likewise forms, together with the carbon atoms C^{α} and C^{β} of a ether group of formula (6), a cycloalkyl radical having from 5 to 7 ring carbon atoms to which a further cycloalkyl radical having from 5 to 7 ring carbon atoms may have been fused, or is a cycloalkyl radical having from 5 to 7 ring carbon atoms to which a further cycloalkyl radical having from 5 to 7 ring carbon atoms may have been fused; and
[G] is a structural unit selected from a single bond and the groups of the formulae 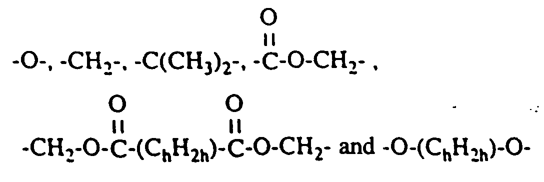 and
h is an integer from 1 to 6, especially from 2 to 4.

11. A compound according to claim 10 having the formula 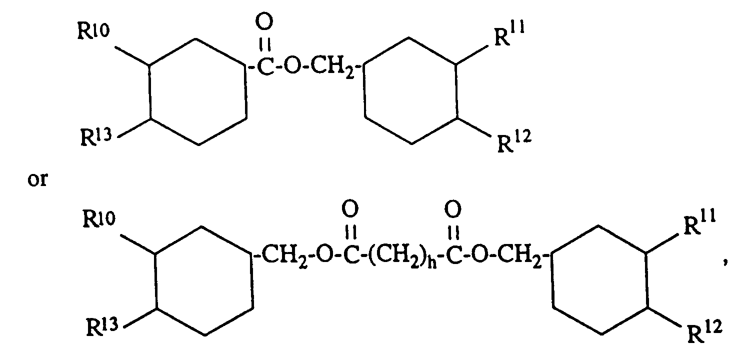 wherein one of the radicals R¹⁰ and R¹³ is a group of the formula H₂C=CH-O-(CH₂)ₓ-O- and the other is a group of the formula R²O- and, likewise, one of the radicals R¹¹ and R¹² is a group of the formula H₂C=CH-O-(CH₂)ₓ-O- and the other is a group of the formula R²O-, and x and h are each independently of the other an integer from 2 to 4.

12. A compound according to claim 1 to 11 wherein
R² is

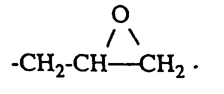

13. A compound according to claim 1 wherein
A is a radical of formula (3) or (4), and
s is an integer from 2 to 4,
t is an integer from 0 to 2,
u is the number 1,
v is the number 0 or 1, and
x and y are each an integer from 2 to 10.

14. A compound according to claim 1 or 13 wherein
A is a radical of formula (4),
R¹⁴ and R¹⁵ are each a hydrogen atom and
z is the number 1.

15. A compound according to claim 1 or 13 having the formula

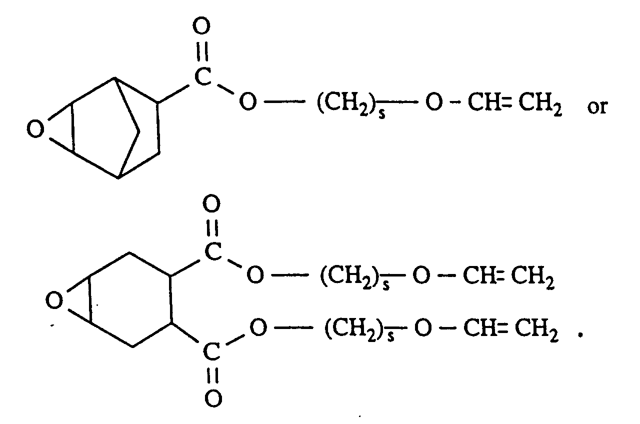

16. The use of a vinyl ether compound according to claim 1 to 15 as a polymerisable component of compositions curable by radiation.

17. A composition comprising one or more vinyl ether compounds according to claim 1 to 16, at least one polymerisable compound that is different therefrom, and at least one photoinitiator for the cross-linking of the compounds.

18. A composition according to claim 17 comprising
5 to 60 % by weight of one or more of the vinyl ether compounds according to claim 1 to 15;
0 to 40 % by weight of mono-, di- or poly-functional acrylates or methacrylates;
30 to 80 % by weight of di- or poly-functional epoxy compounds;
0 to 5 % by weight of radical photoinitiators;
0.5 to 5 % by weight of cationic photoinitiators;
0 to 40 % by weight of hydroxy-terminated polyethers or polyesters; and
0 to 10 % by weight of one or more additives.

19. A method of producing a cured product in which a composition according to either claim 17 or claim 18 is treated with actinic radiation.

20. A method of producing three-dimensional objects from a composition according to either claim 17 or claim 18 by means of stereolithography, comprising a step in which the surface of a layer of the composition is irradiated over its entire surface or in a predetermined pattern with an UV/VIS light source, so that in the irradiated regions a layer of the desired thickness is solidified, and then a fresh layer of the composition is formed on the solidified layer, and that fresh layer is likewise irradiated over its entire surface or in a predetermined pattern, and as a result of the repeated coating and irradiation there are obtained three-dimensional objects consisting of a number of solidified layers adhering one to another.

## Revendications

1. Composé chimique présentant au moins un groupe éther vinylique, lequel composé présente encore au moins un autre groupe fonctionnel pris parmi les groupes acrylate, méthacrylate, époxyde, alcényle, cycloalcényle ainsi que vinylaryle dans la molécule
caractérisé
en ce qu'il s'agit d'un composé de formule suivante
[H₂C=CH―O⁆_{z}A
les symboles figurant dans cette formule et les formules ci-après possèdant la signification suivante :
A représente un reste ayant une fonctionnalité z pris parmi les restes des formules (1), (2), (3) et (4) suivantes 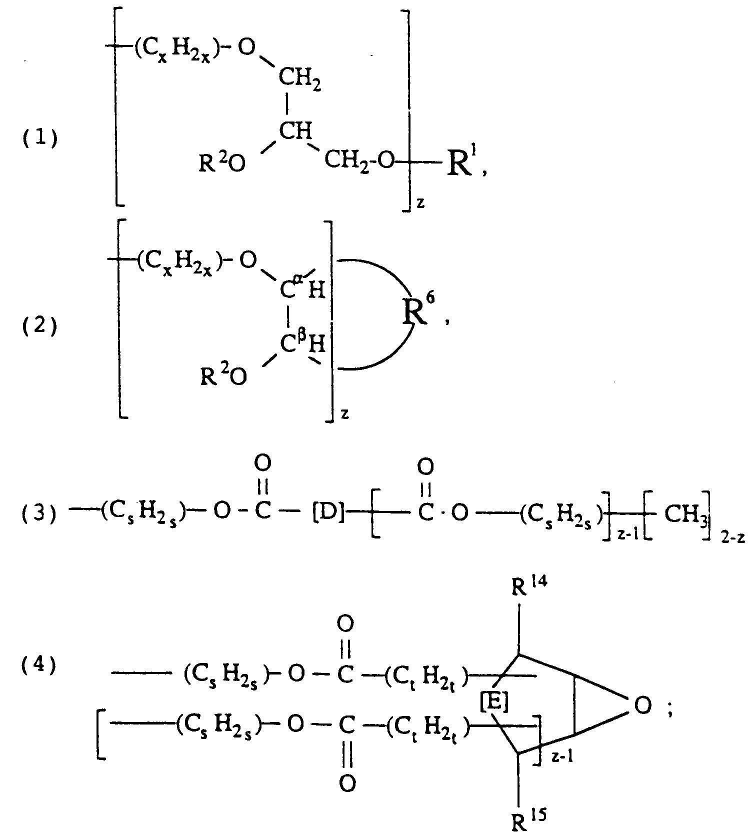
[D] représente un groupe de formule 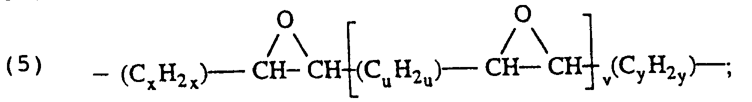
[E] représente un groupe alkylène en C₁ ou un groupe alkylène en C₂ ;
R⁰ représente un atome d'hydrogène ou un groupe méthyle ;
R¹ représente un reste ayant une fonctionnalité z pris parmi les restes
aliphatiques, cyclo-aliphatiques, aromatiques, aliphatiques-aromatiques et aliphatiques-cycloaliphatiques ainsi que les restes polyoxyalkylène ;
R² représente un reste pris parmi les restes de formules 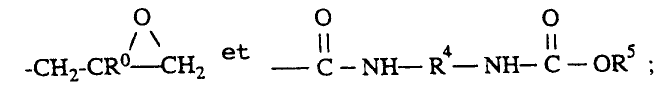
R⁴ représente un groupe pris parmi les groupes de formules 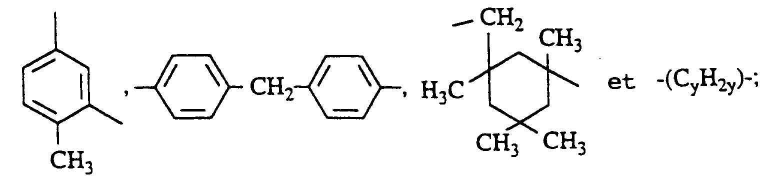
R⁵ représente un groupe pris parmi les groupes de formules 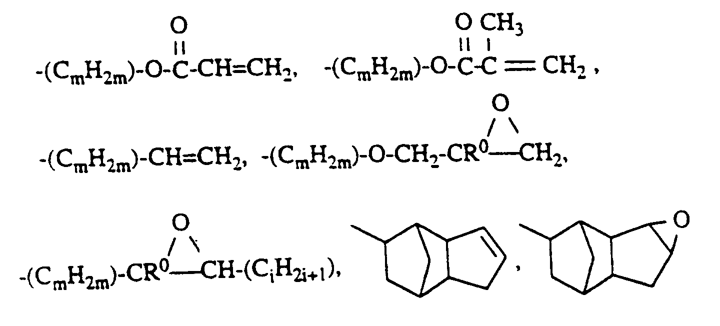 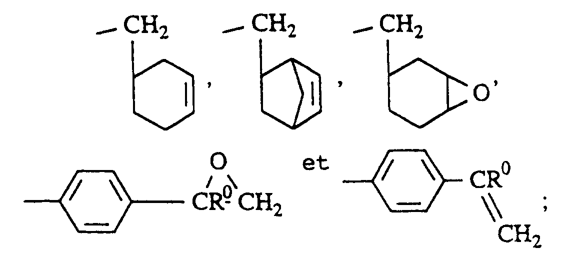
R⁶ représente un groupe organique ayant une fonctionnalité (2·z) pouvant former à chaque fois avec les atomes de carbone C^{α} et C^{β} de chacun des z groupes de formule 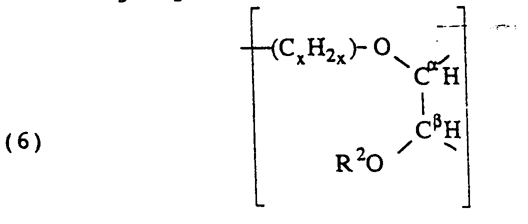 dans un reste de formule (2), un cycle cycloaliphatique présentant au moins 5 atomes de carbone ;
R¹⁴ et R¹⁵ représentent chacun un atome d'hydrogène ou, dans le cas où [E] représente un groupe alkylène en C₂, chacun représente un atome d' hydrogène ou ensemble un groupe méthylène ;
i est un entier de 0 à 20 ;
m est un entier de 1 à 20 ;
s est un entier de 2 à 10 ;
t est un entier de 0 à 10 ;
u dans les différents motifs de formule 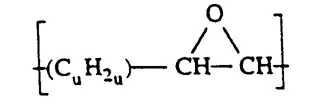 dans la formule (5) représente à chaque fois indépendamment un entier de 1 à 20 ;
v est un entier de 0 à 4 :
x ainsi que y sont chacun, indépendamment l'un de l'autre, un entier de 2 à 20 ;
z est un nombre valant 1 ou 2.

2. Composé selon la revendication 1 de formule 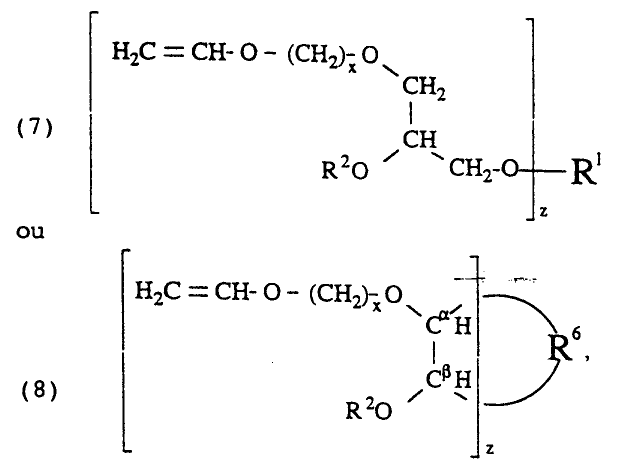 où R¹, R², R⁶, x, z possèdent la même signification que celle donnée à la revendication 1.

3. Composés selon la revendication 1 ou 2, où
R¹ représente un reste ayant une fonctionnalité z, pris parmi
a) des restes aliphatiques comportant de 2 à 20 atomes de carbone,
b) des restes cycloaliphatiques ainsi qu'aromatiques comportant chacun de 6 à 14 atomes de carbone,
c) des restes aliphatiques-aromatiques ainsi que aliphatiques-cycloaliphatiques comportant chacun de 7 à 25 atomes de carbone et
d) des restes polyoxyalkylène de formules
R⁷-[OC_{g}H_{2g}]ₙ- et -(C_{g}H_{2g})-[OC_{g}H_{2g}]ₙ₋₁-,
où
R⁷ représente un groupe alkyle comportant de 1 à 8 atomes de carbone,
g est un nombre de 1 à 8 conformément au nombre moyen d'atomes de carbone d'un motif alkyle du reste polyoxyalkylène et
n est un entier de 2 à 20,
et le reste R¹ est non substitué ou présente de plus un ou plusieurs substituants lesquels,
dans le cas d'un reste aliphatique R¹, sont pris parmi
les substituants alkoxy en C₁-C₄ ou halogène ;
et
dans le cas des restes R¹ d'un autre type, sont pris parmi
les substituants alkyle en C₁-C₄, alkoxy en C₁-C₄ et halogène.

4. Composés selon la revendication 3, où
R¹ représente un reste pris parmi les restes de formules 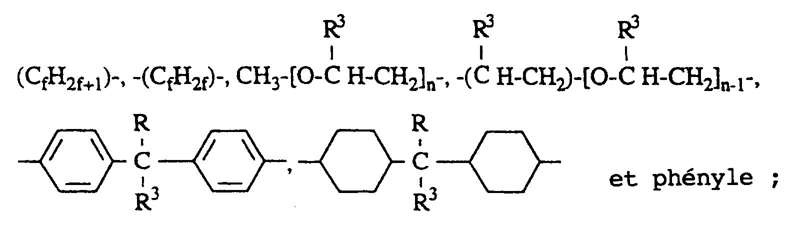 ainsi que
R et R³ représentant chacun, indépendamment l'un de l'autre, un atome d'hydrogène ou un groupe méthyle et
f est un entier de 2 à 20.

5. Composés selon la revendication 4, où
R¹ représente un reste pris parmi les restes de formules 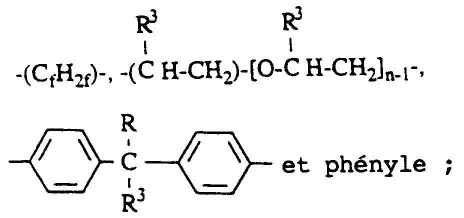
R⁴ représente un groupe pris parmi les groupes de formules 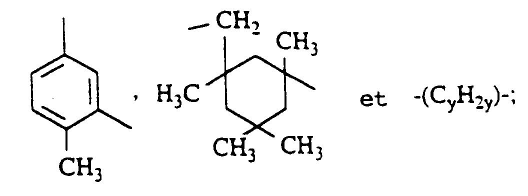
R⁵ représente un groupe pris parmi les groupes de formules 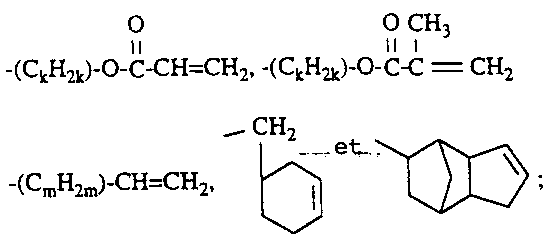 ainsi que
R et R³ les deux, représentent soit un atome d'hydrogène, soit un groupe méthyle ;
k est un entier de 2 à 10,
et à l'indice
m s'applique une limite supérieure de 10.

6. Composés selon la revendication 5, où
R¹ représente un reste pris parmi un reste alkylène comportant de 2 à 4 atomes de carbone, un reste phényle et un reste de formule 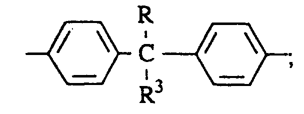
R⁴ représente un groupe de formule 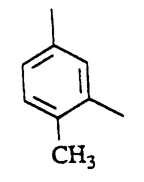 et
R⁵ représente un groupe pris parmi les groupes de formules

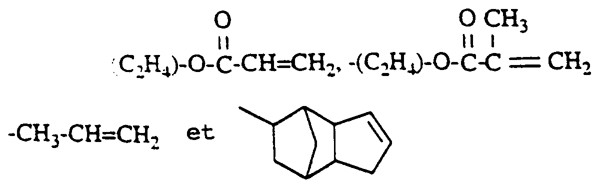

7. Composés selon la revendication 1 ou 2, où
R⁶ représente un groupe organique présentant de 3 à 50 atomes de carbone.

8. Composés selon la revendication 7, où
z vaut 1 et
R⁶ forme, conjointement avec les atomes de carbone C^{α} et C^{β} du groupe de formule (6), un reste cycloalkyle comportant de 5 à 7 atomes de carbone nucléaires,
sur lequel est éventuellement condensé un autre reste cycloalkyle avec 5 à 7 atomes de carbone nucléaires.

9. Composés selon la revendication 8, où
R⁶ forme, conjointement avec les atomes de carbone C^{α} et C^{β} du groupe de formule (6), un reste cyclopentyle ou cyclohexyle.

10. Composés selon la revendication 7, où
R⁶ représente un groupe de formule
R⁸-[G]-R⁹,
où
R⁸ forme, conjointement avec les atomes de carbone C^{α} et C^{β} d'un groupe de formule (6), un reste cycloalkyle présentant 5 à 7 atomes de carbone nucléaires, sur lequel est éventuellement condensé un autre reste cycloalkyle présentant 5 à 7 atomes de carbone nucléaires ;
R⁹ forme, soit avec les atomes de carbone C^{α} et C^{β} également d'un autre groupe de formule (6), un reste cycloalkyle présentant 5 à 7 atomes de carbone nucléaires, sur lequel est éventuellement condensé un autre reste cycloalkyle présentant 5 à 7 atomes de carbone nucléaires,
soit un reste cycloalkyle présentant de 5 à 7 atomes de carbone nucléaires sur lequel est éventuellement condensé un autre reste cycloalkyle présentant de 5 à 7 atomes de carbone nucléaires ; et
[G] représente un élément de structure pris parmi une liaison simple et les groupes de formule 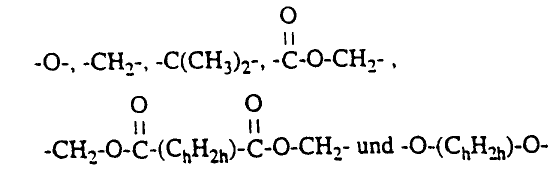 ainsi que
h est un entier de 1 à 6, en particulier de 2 à 4.

11. Composé selon la revendication 10 de formule 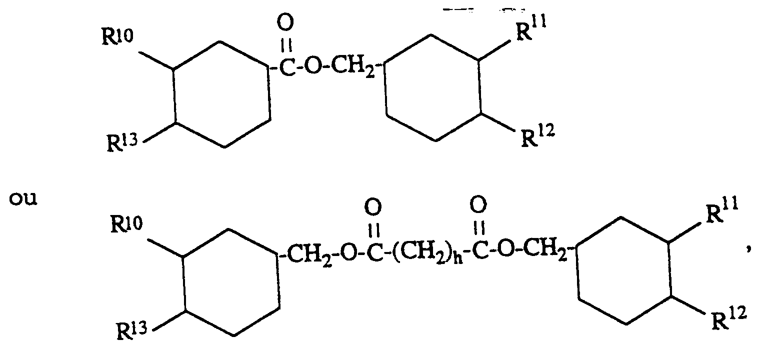 où l'un des restes R¹⁰ ou R¹³ représente un groupe de formule H₂C=CH-O-(CH₂)ₓ-O- et l'autre représente un groupe de formule R²O- et de même, l'un des restes R¹¹ ou R¹² représente un groupe de formule H₂C=CH-O-(CH₂)ₓ-O- et l'autre un groupe de formule R²O-, x et h représentant chacun, indépendamment l'un de l'autre, un nombre entier de 2 à 4.

12. Composés selon la revendication 1 à 11, où
R² représente

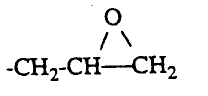

13. Composés selon la revendication 1, où
A représente un reste de formule (3) ou (4),
s représente un entier de 2 à 4,
t représente un entier de 0 à 2,
u vaut 1,
v vaut 0 ou 1, et
x ainsi que y représentent un entier de 2 à 10.

14. Composés selon la revendication 1 ou 13, où
A représente un reste de formule (4),
R¹⁴ et R¹⁵ représentent chacun un atome d'hydrogène, ainsi que
z vaut 1.

15. Composé selon la revendication 1 ou 13 répondant à la formule

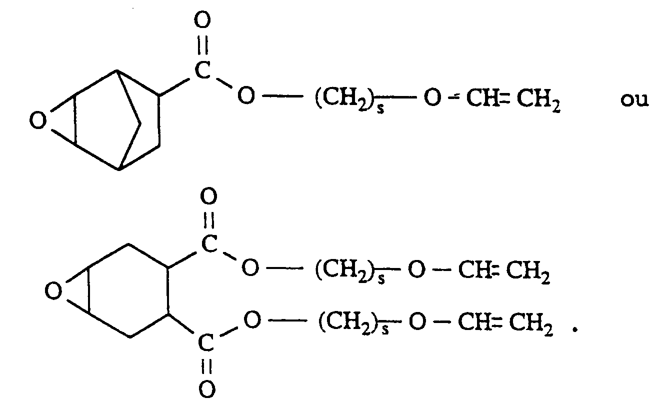

16. Utilisation des composés d'éthers vinyliques selon la revendication 1 à 15 en tant qu'un composant polymérisable de compostions durcissables par irradiation.

17. Composition contenant un ou plusieurs composés d'éthers vinyliques selon la revendication 1 à 16, au moins un composé polymérisable différent de ceux-ci et au moins un photoamorceur pour la réticulation des composés.

18. Composition selon la revendication 17, contenant
de 5 à 60% massiques d'un ou plusieurs composés d'éthers vinyliques selon les revendications 1 à 15,
de 0 à 40% massiques d'acrylates ou de méthacrylates mono-, di- ou multifonctionnels ;
de 30 à 80% massiques de composés époxydiques di- ou polyfonctionnels ;
de 0 à 5% massiques de photoamorceurs radicalaires ;
de 0,5 à 5% massiques de photoamorces cationiques
de 0 à 40% massiques de polyéthers ou polyesters présentant des groupes terminaux hydroxyle ; et
de 0 à 10% massiques d'un ou plusieurs additifs.

19. Procédé de préparation d'un produit durci, dans lequel on traite une composition selon l'une des revendications 17 ou 18 par un rayonnement actinique.

20. Procédé de préparation d'objets tridimensionnels à partir d'une composition selon l'une des revendications 17 ou 18 par stéréolithographie, comprenant une étape dans laquelle la surface d'une couche de la composition est irradiée par une source de lumière UV/VIS sur la totalité de la surface ou selon un modèle déterminé auparavant de façon telle que dans les régions irradiées une couche d'une épaisseur de couche voulue est solidifiée, puis une nouvelle couche de la composition se forme sur la couche solidifiée, laquelle également est irradiée sur la totalité de la surface ou selon un modèle déterminé auparavant, et en obtenant par revêtement et irradiation répétés des objets tridimensionnels constitués par plusieurs couches solidifiées adhérentes les unes aux autres.
